# EUROPEAN PATENT SPECIFICATION

(11) **EP 4 059 492 B1**
(45) Date of publication and mention of the grant of the patent: **17.04.2024**
(21) Application number: 22172044.4
(22) Date of filing: 16.12.2016
(51) Int. Cl.: A61K 9/16, A61K 38/17, A61K 9/50

(54) **COMPOSITIONS AND METHODS OF MANUFACTURING PROTEIN MICROPARTICLES**
ZUSAMMENSETZUNGEN UND VERFAHREN ZUM HERSTELLEN VON PROTEINMIKROPARTIKELN
COMPOSITIONS ET PROCÉDÉS DE FABRICATION DE MICROPARTICULES PROTÉIQUES

(30) Priority: 16.12.2015 US 201562268259 P
(43) Date of publication of application: 21.09.2022
(62) Divisional of application: 16820541.7
(73) Proprietor: Regeneron Pharmaceuticals, Inc., Tarrytown, NY 10591 (US)
(72) Inventor: BRUDNICKI, Philip, New York, 10028 (US); CHEN, Hunter, New York, 10591 (US)
(74) Representative: J A Kemp LLP

(56) References cited:
- US-A- 6 019 968
- US-A1- 2007 298 116
- US-B1- 6 284 282
- AJMERA ANKUR ET AL: "Stabilisation of proteins via mixtures of amino acids during spray drying", INTERNATIONAL JOURNAL OF PHARMACEUTICS, vol. 463, no. 1, 8 January 2014 (2014-01-08), pages 98-107, XP028605165, ISSN: 0378-5173, DOI: 10.1016/J.IJPHARM.2014.01.002
- JAIN R ET AL: "CONTROLLED DRUG DELIVERY BY BIODEGRADABLE POLY(ESTER) DEVICES: DIFFERENT PREPARATIVE APPROACHES", DRUG DEVELOPMENT AND INDUSTRIAL PHARMACY, NEW YORK, NY, US, vol. 24, no. 8, 1 January 1998 (1998-01-01), pages 703-727, XP009052301, ISSN: 0363-9045, DOI: 10.3109/03639049809082719

## Description

### FIELD

The invention generally pertains to compositions and methods of formulating proteins stable over an extended period of time. The invention specifically pertains to compositions and methods of making therapeutic protein formulations that remain stable and biologically active at ambient and physiological temperatures for an extended period of time.

### BACKGROUND

Therapeutic macromolecules, such as antibodies and receptor Fc-fusion proteins, must be formulated in a manner that not only makes the molecules suitable for administration to patients, but also maintains their stability during storage and while at the site of administration. For example, therapeutic proteins *(e.g.,* antibodies) in liquid solution are prone to degradation, aggregation and/or undesired chemical modifications unless the solution is formulated properly. Considerations aside from stability must also be taken into account when preparing a therapeutic protein formulation. Examples of such additional considerations include the viscosity of the solution and the concentration of antibody that can be accommodated by a given formulation. When formulating a therapeutic protein for extended release, great care must be taken to arrive at a formulation that remains stable over time and at storage and physiological temperature, contains an adequate concentration of antibody or other therapeutic biological, and possesses other properties which enable the formulation to be conveniently administered to patients.

Liquid formulations of biological molecules are generally designed to provide long term stability when frozen or refrigerated, but often fail to provide long term stability at room temperature. One solution known in the art to preserve stability and biological/therapeutic activity of a biological molecule is to freeze-dry or otherwise lyophilize the molecule. Lyophilization can provide a dry "cake" that remains relatively stable at ambient temperature for a relatively long period of time. Room temperature stability may be especially important in storing and distributing biotherapeutics around the world, especially in places where electricity and refrigeration are not reliable.

Another emerging issue in the pharmaceutical formulation arts is the need for increased concentrations of therapeutic protein to facilitate the delivery of large amounts of drug in a small amount of space. The problem of maximizing the amount of protein drug per unit volume is exacerbated by the reciprocal need to reduce the amount of excipients that help to stabilize the protein. As the molar ratio of protein drug to stabilizer increases, the amount of protein is maximized at the risk of destabilizing the protein.

U.S. Patent Application Publication No. 2016/0176986 A1 describes a high concentration (≥ 200m mg/mL) IgG1 formulation in a non-aqueous solvent. That formulation contains spray-dried IgG1 particles suspended in a non-aqueous solution designed for subcutaneous delivery. The spray-dried particles contain trehalose as a stabilizer and the IgG1 molecule in a weight-to-weight ratio of 1:2.

Shire et al. (J. Pharm. Sci., vol 93, no. 6, June 2004, 1390-1402) describe the development of high concentration protein formulations by lyophilization. Shire describes the optimum molar ratio of lyoprotectant to protein as 300:1 or greater. An antibody formulation with a molar ratio of 500:1 lyoprotectant to antibody was described as having significant stability at room temperature, but with undesirable hypertonicity. That same antibody formulated with lower lyoprotectant (250:1 lyoprotectant to antibody by mole) showed improved and useful tonicity, but with much reduced stability. The compromise 250:1 formulation requires storage at 2-8°C to maintain reasonable stability.

Ajmera and Scherliesz (Int. J. Pharm., vol. 463, 2014, 98-107) describe the use of nitrogen dense amino acids arginine and histidine to stabilize catalase during spray drying. The stabilizing effect was attributed to nitrogen mediated hydrogen bonding. The weight-to-weight ratio of amino acid to catalase for effective stabilization was 1:1 or 2:1. Patent document US 6,284,282 B1 discloses a method of preparing a spray-freeze dried composition for pulmonary administration comprising particles of a therapeutic protein.

### SUMMARY

In one aspect, the invention provides a method of manufacturing a formulated pharmaceutical powder according to claim 1. According to this aspect, an aqueous solution containing a thermal stabilizer and a glycoprotein with a mass ratio at or between 1:5-2:5 is atomized. Heat is then applied to the atomized aqueous solution to evaporate the water from the aerosolized droplets and form a protein powder. In one embodiment, individual particles comprising the protein powder are subsequently coated with a biodegradable polymer.

In one aspect, the invention provides a method of manufacturing a formulated pharmaceutical powder according to claim 6. According to this aspect, an aqueous solution containing a thermal stabilizer and a glycoprotein with a molar ratio of less than 300:1 is atomized. Heat is then applied to the atomized aqueous solution to evaporate the water from the aerosolized droplets and form a protein powder. In one embodiment, individual particles comprising the protein powder are subsequently coated with a biodegradable polymer.

Disclosed , but not claimed, is a method of manufacturing a formulated pharmaceutical powder. According to this aspect, an aqueous solution containing a glycoprotein without a thermal stabilizer is atomized. Heat is then applied to the atomized aqueous solution to evaporate the water from the aerosolized droplets and form a protein powder. In one embodiment, individual particles comprising the protein powder are subsequently coated with a biodegradable polymer.

In one aspect, the invention provides a formulated pharmaceutical powder according to claim 11 containing 60%-97% (w/w) of a glycoprotein, and 3%-40% (w/w) of a thermal stabilizer According to this aspect, the formulated pharmaceutical powder is not bone dry, and the percent change in the amount of high molecular weight species of the glycoprotein is less than 5%. In one embodiment, individual protein particles of the powder are have a biodegradable polymer coating.

### FIGURES

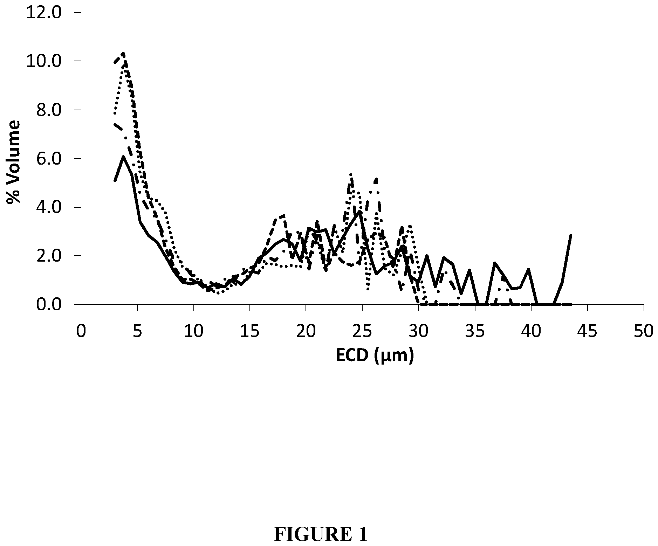

Figure 1 is a line plot depicting the size distribution of particles in Equivalent Circular Diameter (ECD) by volume of spray dried particles suspended in ethanol as measured by Microflow Imaging (MFI). The particles were spray dried at different inlet temperatures: 100°C (dash-dot line); 110°C (dashed line); 120°C (dotted line); and 130°C (solid line).

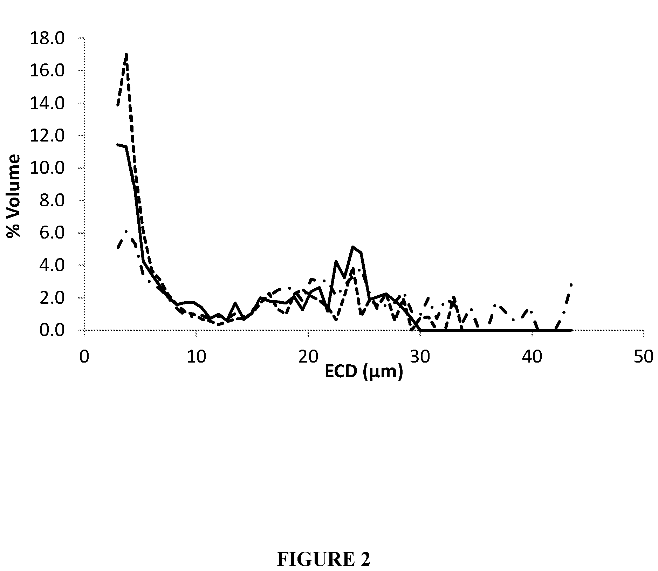

Figure 2 is a line plot depicting the size distribution in Equivalent Circular Diameter (ECD) by volume of spray dried particles suspended in ethanol as measured by Microflow Imaging (MFI). The pre-spray dried formulation contained 0% (dash-dot line), 0.03% (dashed line), and 0.1% (solid line) w/v polysorbate 20 (PS20).

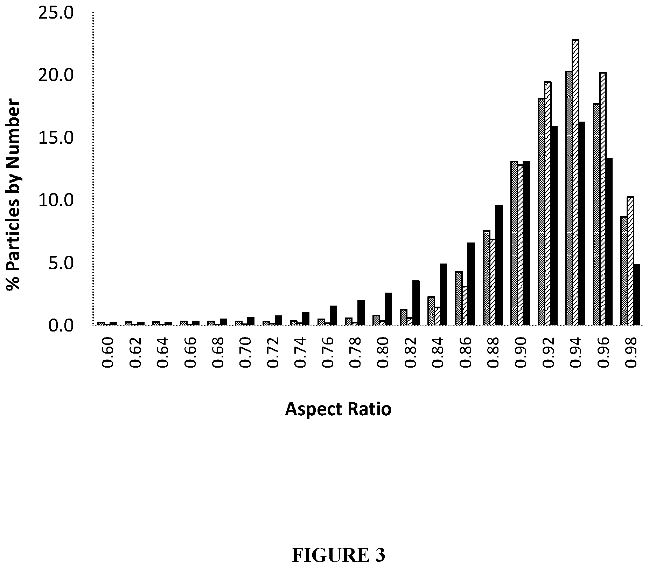

Figure 3 is a histogram depicting the number distribution of spray dried particles by aspect ratio suspended in ethanol as measured by Microflow Imaging (MFI). An aspect ratio equal to 1 represents a spherical particle morphology. The pre-spray dried formulation contained 0% (stipple filled histogram), 0.03% (diagonal hatch filled histogram), and 0.1% (solid filled histogram)w/v polysorbate 20 (PS20).

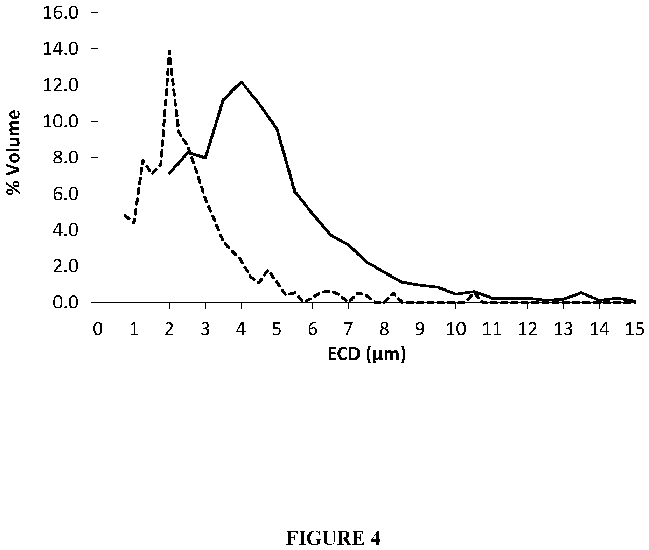

Figure 4 is a line graph depicting size distribution in Equivalent Circular Diameter (ECD) by volume of spray dried particles suspended in ethanol as measured by Microflow Imaging (MFI). The low solute process (depicted by dashed line) used about 10-fold lower solute concentration than the standard process (depicted by solid line).

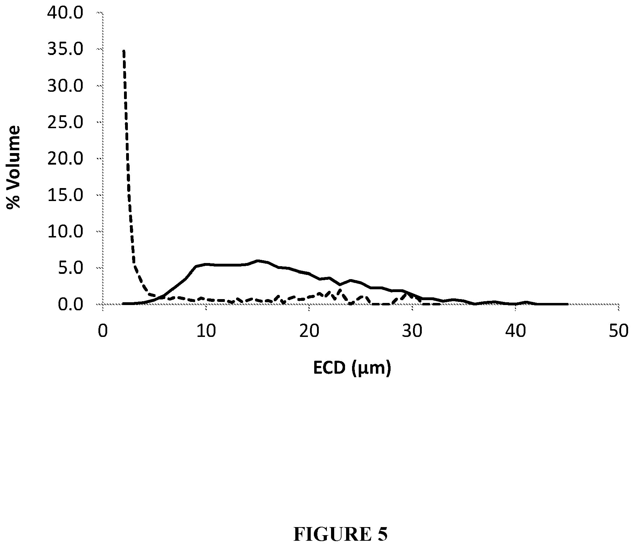

Figure 5 is a line graph depicting size distribution in Equivalent Circular Diameter (ECD) by volume of spray dried particles (protein microparticles without polymer coating; dashed line) and spray coated particles (polymer coated protein microparticles; solid line) as measured by Microflow Imaging (MFI).

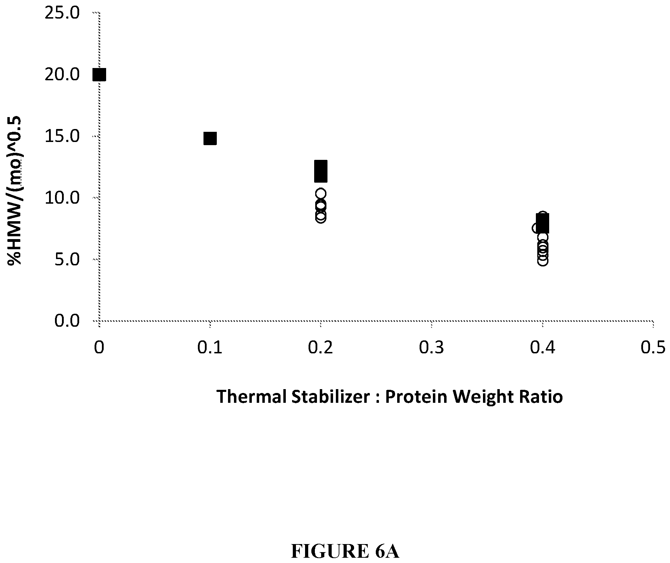

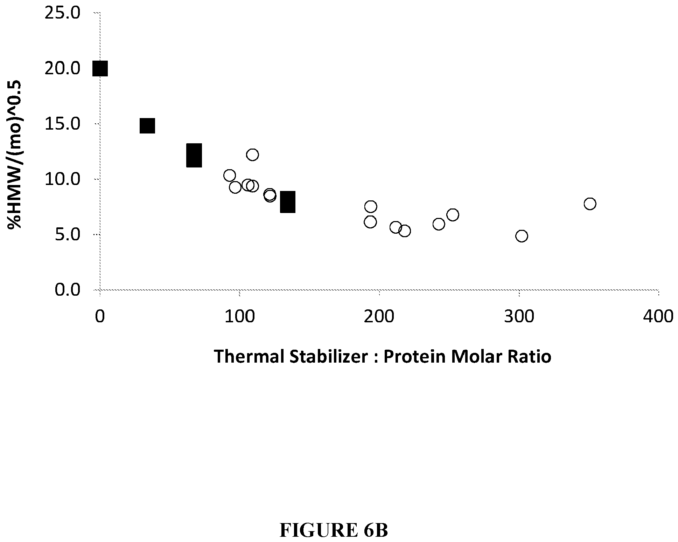

Figure 6, panels A and B are dot plots depicting the rate of high molecular weight species (HMW) formation as function of square root of time at 50°C as measured by SEC-UPLC. The rates are plotted according to their thermal stabilizer : protein weight ratio (6A) and molar ratios (6B). Formulations denoted by the solid filled squares contain sucrose only. Formulations containing other thermal stabilizers are depicted by open circles.

### DETAILED DESCRIPTION

Although any methods and materials similar or equivalent to those described herein can be used in the practice or testing of the present invention, the preferred methods and materials are now described.

An emerging problem in the biopharmaceutical industry is the provision of stable protein formulations at a high enough concentrations to enable the delivery of an effective dose in a small volume. To help maintain protein stability, various stabilizers and other excipients are included in the formulation. This presents a trade-off between protein stability and protein concentration. The invention provides an improved formulation containing a protein that remains stable at high density. The amount of stable protein delivered per unit volume is increased without sacrificing protein stability.

In one aspect, the invention provides a formulated pharmaceutical powder that contains about 60%-97% (w/w) of a glycoprotein, and about 3%-40% (w/w) of a thermal stabilizer. In another aspect, the invention provides a formulated pharmaceutical powder that contains about 85%-97% (w/w) of a glycoprotein without a thermal stabilizer. In one embodiment of that aspect, residual water that remains in the powder stabilizes the protein.

In some embodiments, the formulated pharmaceutical powder contains 60-70% (w/w) glycoprotein, 60-70% (w/w) glycoprotein, 65-75% (w/w) glycoprotein, 70-80% (w/w) glycoprotein, 75-85% (w/w) glycoprotein, 80-90% (w/w) glycoprotein, 85-95% (w/w) glycoprotein, about 60% (w/w) glycoprotein, about 62% (w/w) glycoprotein, about 64% (w/w) glycoprotein, about 66% (w/w) glycoprotein, about 68% (w/w) glycoprotein, about 70% (w/w) glycoprotein, about 72% (w/w) glycoprotein, about 74% (w/w) glycoprotein, about 76% (w/w) glycoprotein, about 78% (w/w) glycoprotein, about 80% (w/w) glycoprotein, about 82% (w/w) glycoprotein, about 84% (w/w) glycoprotein, about 86% (w/w) glycoprotein, about 88% (w/w) glycoprotein, about 90% (w/w) glycoprotein, about 92% (w/w) glycoprotein, about 94% (w/w) glycoprotein, about 96% (w/w) glycoprotein or about 98% (w/w) glycoprotein.

In some embodiments, the formulated pharmaceutical powder contains 3-6% (w/w) thermal stabilizer, 5-7% (w/w) thermal stabilizer, 6-8% (w/w) thermal stabilizer, 7-9% (w/w) thermal stabilizer, 8-10% (w/w) thermal stabilizer, 9-11% (w/w) thermal stabilizer, 10-12% (w/w) thermal stabilizer, 11-13% (w/w) thermal stabilizer, 12-14% (w/w) thermal stabilizer, 13-15% (w/w) thermal stabilizer, 14-16% (w/w) thermal stabilizer, 15-17% (w/w) thermal stabilizer, 16-18% (w/w) thermal stabilizer, 17-19% (w/w) thermal stabilizer, 18-20% (w/w) thermal stabilizer, 19-21% (w/w) thermal stabilizer, 20-22% (w/w) thermal stabilizer, 21-23% (w/w) thermal stabilizer, 22-24% (w/w) thermal stabilizer, 23-25% (w/w) thermal stabilizer, 24-26% (w/w) thermal stabilizer, 25-27% (w/w) thermal stabilizer, 22-24% (w/w) thermal stabilizer, 23-25% (w/w) thermal stabilizer, 24-26% (w/w) thermal stabilizer, 25-27% (w/w) thermal stabilizer, 22-24% (w/w) thermal stabilizer, 23-25% (w/w) thermal stabilizer, 24-26% (w/w) thermal stabilizer, 25-27% (w/w) thermal stabilizer, 26-28% (w/w) thermal stabilizer, 27-29% (w/w) thermal stabilizer, 28-30% (w/w) thermal stabilizer, 29-31% (w/w) thermal stabilizer, 30-32% (w/w) thermal stabilizer, 31-33% (w/w) thermal stabilizer, 32-34% (w/w) thermal stabilizer, 33-35% (w/w) thermal stabilizer, 34-36% (w/w) thermal stabilizer, 35-37% (w/w) thermal stabilizer, 36-38% (w/w) thermal stabilizer, 37-39% (w/w) thermal stabilizer, 38-40% (w/w) thermal stabilizer or 39-41% (w/w) thermal stabilizer.

In some embodiments, the formulated pharmaceutical powder of the invention contains a population of micron-scale protein particles. The powder-constituent micron-scale protein particles may be referred to herein as "micronized protein-containing particles," "protein particles," "protein microparticles," "microparticles," "population of micronized protein-containing particles," "population of protein particles," "population of protein microparticles," "population of microparticles," "powder-constituent protein microparticles," or "constituent protein microparticles."

In some embodiments, the subject formulated powder is free flowing under routine storage conditions and during pharmaceutical filling operations. In some embodiments, the subject powder has a Hausner ratio *(i.e.,* the ratio of the tapped density of powder over the bulk density of powder) under filling operation conditions or storage conditions of < 1.5, < 1.45, < 1.4, < 1.35, < 1.3, <1.25, < 1.2, < 1.15 or <1.1.

In some embodiments, the preferred method of determining the flowability of the powder is by a tumbling vial assay. A clear glass vial is partially filled with the formulated pharmaceutical powder, then the vial is rotated on its vertical axis. A powder that is able to freely tumble while rotating the vial is flowable. A powder that has some amount of static charge causing it to adhere to vial walls or requires slight tapping or static removal in order to make it tumble while rotating the vial is considered flowable in some embodiments. In some embodiments, the powder is flowable where < 30%, < 25%, < 20%, < 15%, < 10% or < 5% of the vial inner surface is covered with powder after rotation of the vial. In some embodiments, the powder is flowable where constituent particles that adhered to the walls of the vial after rotation are removed from the walls of the vial when the vial is tapped on a hard surface or with a finger 1 time, 2 times, 3 times, 4 times, 5 times, 6 times, 7 times, 8 times, 9 times or 10 times. In some embodiments, the powder is flowable where constituent particles that adhered to the walls of the vial after rotation are removed from the walls of the vial when the vial is tapped with a cumulative force of ≤ 25 micronewtons (µN), about 25 µN, about 20 µN, about 15 µN, about 10 µN, about 9 µN, about 8 µN, about 7 µN, about 6 µN, about 5 µN, about 4 µN, about 3 µN, about 2 µN, about 1 µN, or < 1 µN. In some embodiments, a powder where the vast majority of the constituent particles adhere to vials walls due to static forces and will not tumble is not flowable. In some embodiments, a preferred reference powder that represents a flowable powder consists of 50-150 µm glass beads (Malvern QA standard, part # CRM0016, produced by Whitehouse Scientific, Chester, UK.)

In some embodiments, powder flowability is measured by angle of repose, compressibility *(e.g.,* Hausner Ratio), flow in a rotating drum, flow through an orifice, shear cell analysis, rheometry, or rate of dispensing. In some embodiments, flowability is measured with a REVOLUTION Powder Analyzer (Mercury Scientific Inc., Newtown, CT), EVOLUTION Powder Tester (Mercury), VOLUTION Powder Flow Tester (Mercury), or FT 300 Flowability Tester (Sotax AG, Aesch, CH.) Rao et al., European Journal of Pharmaceutics and Biopharmaceutics, Volume 74, Issue 2, February 2010, Pages 388-396 is mentioned herein for flow rate determination by drum rotewation and avalanching.

In some embodiments, flowability is determined by flow through an orifice. In some embodiments where amount of powder is limiting *(e.g.,* 1-2 grams or less), flow through an orifice is performed by iterative breaking of vault structures followed by flowing the powder through an orifice *(e.g.,* 3 mm) and measuring the weight of the powder that passes through the orifice as a function of time. Flow rate is reported in milligrams per second. In some embodiments, the formulated pharmaceutical powder is flowable when the flow rate through a 3 mm orifice is ≥ 10 mg/sec, ≥ 15 mg/sec, ≥ 20 mg/sec, ≥ 25 mg/sec, ≥ 30 mg/sec, ≥ 35 mg/sec, ≥ 40 mg/sec, ≥ 45 mg/sec, ≥ 50 mg/sec, ≥ 55 mg/sec, ≥ 60 mg/sec, ≥ 65 mg/sec, ≥ 70 mg/sec, ≥ 75 mg/sec, ≥ 80 mg/sec, ≥ 85 mg/sec, ≥ 90 mg/sec, ≥ 95 mg/sec or ≥ 100 mg/sec. Seppälä et al., AAPS PharmSciTech, Vol. 11, No. 1, March 2010, Pages 402-408 is mentioned herein for characterization of drug-excipient blend flowability by flow rate through an orifice.

The component protein microparticle of the subject formulated pharmaceutical powder can be roughly spherical in shape. Some protein microparticles approach sphericity, while other protein microparticles have a more irregular shape. The shape of the protein microparticles may be determined by inter alia static light scattering (DLS), micro-flow imaging (MFI), or laser diffraction. DLS relies upon measuring the intensity of scattered light at several different angles resulting from intense light directed at a particle suspension. The Fraunhofer equation is then applied to the scatter data to determine size and shape of protein microparticles. US 5,104,221 A is mentioned herein for the application of the Fraunhofer equation to determine the size and shape of micron-scale particles. DLS is generally applied to objects of 1 micron or smaller. MFI is based upon a series of bright field images of particles obtained from a sample stream passing through a flow cell. The images are analyzed to determine size of particle and circularity or aspect ratio. Circularity refers to how round or spherical the protein microparticle is, and is expressed on a scale of 0-1, where 1 is perfectly spherical. The term "aspect ratio", which usually connotes length by width of an object, is used interchangeably with "circularity". Aspect ratio may be expressed as the ratio of the length of the minor axis to the length of the major axis. Thus, a more spherical particle has an "aspect ratio" closer to 1 (*e.g*., 0.98).

Laser diffraction is another Fraunhofer diffraction-based method used to determine particle shape and size in the 1-100 micron range. Lasered light is passed through the particle suspension and an intervening lens focusses the diffracted light onto a sensor. De Boer et al., Int. J. Pharmaceutics, 249 (1-2): 219-231 (2002) is mentioned herein for particle sizing by laser diffraction. In one embodiment, the size distribution of the powder constituent protein microparticle is determined by laser diffraction using a Malvern MASTERSIZER 3000 laser particle size analyzer (Malvern, UK.)s

In some embodiments, the shape of the protein microparticle is approximately spheroidal. In one embodiment, the aspect ratio of the protein microparticle is ≥ 0.80. In another embodiment, the aspect ratio of the protein microparticle is about 0.90 to about 0.98. The shape of the protein microparticle can be determined inter alia by micro-flow imaging (MFI).

As used herein, the term "diameter" of a powder-component microparticle includes the meaning of any of the following: (a) the diameter of a sphere which circumscribes the microparticle, (b) the diameter of the largest sphere that fits within the confines of the microparticle or the protein core, (c) any measure between the circumscribed sphere of (a) and the confined sphere of (b), including the mean between the two, (d) the length of the longest axis of the microparticle, (e) the length of the shortest axis of the microparticle, (f) any measure between the length of the long axis (d) and the length of the short axis (e), including the mean between the two, and/or (g) equivalent circular diameter ("ECD"), as determined by MFI, light obscuration methods such as DLS, or the like. MFI and DLS are generally described in Sharma et al., "Micro-flow imaging: flow microscopy applied to subvisible particulate analysis in protein formulations," 12(3) AAPS J. 455-64 (2010); and B. J. Frisken, "Revisiting the Method of Cumulants for the Analysis of Dynamic Light-Scattering Data," 40(24) Applied Optics 4087-91 (2001) are mentioned herein for micro-flow imaging and dynamic light scattering. Diameter is generally expressed in micrometers (µm or micron).

The protein microparticle of the subject formulated pharmaceutical powder can be approximately spherical in shape and have a diameter ranging from 2 microns to about 45 microns. In one embodiment, the majority of the protein microparticles of the powder have a diameter less than 10 microns as determined by MFI. In some embodiments, the mode protein microparticle size of the subject formulated pharmaceutical powder is 1-10 µm, 2-10 µm, 3-10 µm, 4-10 µm, 5-10 µm, 6-10 µm, 7-10 µm, 8-10 µm, 9-10 µm, 1-9 µm, 1-8 µm, 1-7 µm, 1-6 µm, 1-5 µm, 1-4 µm, 1-3 µm, 1-2 µm, 2-9 µm, 2-8 µm, 2-7 µm, 2-6 µm, about 1 µm, about 1.5 µm, about 2 µm, about 2.5 µm, about 3 µm, about 3.5 µm, about 4 µm, about 4.5 µm, about 5 µm, about 5.5 µm, about 6 µm, about 6.5 µm, about 7 µm, about 7.5 µm, about 8 µm, about 8.5 µm, about 9 µm, about 9.5 µm or about 10 µm.

In some embodiments, the diameter of the protein microparticle is < 50 µm. In one embodiment, the diameter of the protein microparticle is < 12 µm. In another embodiment, the diameter of the protein microparticle is < 10 µm. In yet another embodiment, the diameter of the protein microparticle is about 0.5 µm to about 7.0 µm. In one specific embodiment, the diameter of the protein microparticle is about 5.0 µm. In another specific embodiment, the diameter of the protein microparticle is about 2.5 µm. Diameter of the protein microparticles can be determined inter alia by MFI or static light scattering.

The diameter of the protein microparticle is positively correlated to the volume of the protein microparticle. For example, a perfect 10 micron sphere is approximately 5×10⁻⁴ nanoliters, and a perfect 5 micron sphere is approximately 7 × 10⁻⁵ nanoliters. In some embodiments, the mode protein microparticle volume of the subject formulated pharmaceutical powder is 5×10⁻⁷ - 5×10⁻⁴ nL, 10⁻⁶ - 5×10⁻⁴ nL, 5×10⁻⁶ - 5×10⁻⁴ nL, 10⁻⁵ - 5×10⁻⁴ nL, 5×10⁻⁵-5×10⁻⁴ nL, 10⁻⁴ - 5×10⁻⁴ nL, about 5×10⁻⁷ nL, about 6×10⁻⁷ nL, about 7×10⁻⁷ nL, about 8×10⁻⁷ nL, about 9×10⁻⁷ nL, about 10⁻⁶ nL, about 2×10⁻⁶ nL or about 3×10⁻⁶ nL, about 4×10⁻⁶ nL, about 5×10⁻⁶ nL or about 6×10⁻⁶ nL, about 7×10⁻⁶ nL, about 8×10⁻⁶ nL or about 9×10⁻⁶ nL, about 10⁻⁵ nL, about 2×10⁻⁵ nL or about 3×10⁻⁵ nL, about 4×10⁻⁵ nL, about 5×10⁻⁵ nL or about 6×10⁻⁵ nL, about 7×10⁻⁵ nL, about 8×10⁻⁵ nL or about 9×10⁻⁵ nL, about 10⁻⁴ nL, about 2×10⁻⁴ nL or about 3×10⁻⁴ nL, about 4×10⁻⁴ nL, about 5×10⁻⁴ nL or about 6×10⁻⁴ nL, about 7×10⁻⁴ nL, about 8×10⁻⁴ nL or about 9×10⁻⁴ nL, or 10⁻³ nL. Disclosed, but not claimed, is that the formulated pharmaceutical powder is "bone dry". A powder that is bone dry may contain up to 3% (w/w) water. In some embodiments, a bone dry powder contains ≤ 3% (w/w) water, ≤ 2.9% (w/w) water, ≤ 2.8% (w/w) water, ≤ 2.7% (w/w) water, ≤ 2.6% (w/w) water, ≤ 2.5% (w/w) water, ≤ 2.4% (w/w) water, ≤ 2.3% (w/w) water, ≤ 2.2% (w/w) water, ≤ 2.1% (w/w) water, ≤ 2.0% (w/w) water, ≤ 1.9% (w/w) water, ≤ 1.8% (w/w) water, ≤ 1.7% (w/w) water, ≤ 1.6% (w/w) water, ≤ 1.5% (w/w) water, ≤ 1.4% (w/w) water, ≤ 1.3% (w/w) water, ≤ 1.2% (w/w) water, ≤ 1.1% (w/w) water, ≤ 1.0% (w/w) water, ≤ 0.9% (w/w) water, ≤ 0.8% (w/w) water, ≤ 0.7% (w/w) water, ≤ 0.6% (w/w) water, ≤ 0.5% (w/w) water, ≤ 0.4% (w/w) water, ≤ 0.3% (w/w) water, ≤ 0.2% (w/w) water or ≤ 0.1% (w/w) water. In some embodiments, a bone dry powder contains about 3% (w/w) water, about 2.5% (w/w) water, about 2% (w/w) water, about 1.5% (w/w) water, about 1% (w/w) water, about 0.5% (w/w) water or about 0% (w/w) water. In some embodiments, a bone dry powder contains 0%-3% (w/w) of water, 0.05%-3% (w/w) of water, 0.5%-3% (w/w) of water, 1%-3% (w/w) of water, 2%-3% (w/w) of water, 0%-2% (w/w) of water, 0.05%-2% (w/w) of water, 0.5%-2% (w/w) of water, 1%-2% (w/w) of water, 0%-1% (w/w) of water, 0.05%-1% (w/w) of water or 0.5%-1% (w/w) of water.

In another embodiment, the provided formulated pharmaceutical powder is not bone dry. In some embodiments, the provided formulated pharmaceutical powder that is not bone dry contains no more than 10% (w/w) water. In some embodiments, the provided formulated pharmaceutical powder that is not bone dry contains more than 3% (w/w) water and no more than 10% (w/w) water, 3.5%-10% (w/w) water, 4%-10% (w/w) water, 4.5%-10% (w/w) water, 5%-10% (w/w) water, 5.5%-10% (w/w) water, 6%-10% (w/w) water, 6.5%-10% (w/w) water, 7%-10% (w/w) water, 7.5%-10% (w/w) water, 8%-10% (w/w) water, 8.5%-10% (w/w) water, 9%-10% (w/w) water, 9.5%-10% (w/w) water, >3%-9% (w/w) water, >3%-9% (w/w) water, >3%-8.5% (w/w) water, >3%-8% (w/w) water, >3%-7.5% (w/w) water, >3%-7% (w/w) water, >3%-6.5% (w/w) water, >3%-6% (w/w) water, >3%-5.5% (w/w) water, >3%-5% (w/w) water, >3%-4.5% (w/w) water, >3%-4% (w/w) water, >3%-3.5% (w/w) water, about 3.1% (w/w) water, about 3.5% (w/w) water, about 4% (w/w) water, about 4.5% (w/w) water, about 5% (w/w) water, about 5.5% (w/w) water, about 6% (w/w) water, about 6.5% (w/w) water, about 7% (w/w) water, about 7.5% (w/w) water, about 8% (w/w) water, about 8.5% (w/w) water, about 9% (w/w) water, about 9.5% (w/w) water or about 10% (w/w) water.

In some embodiments, the provided formulated pharmaceutical powder may contain up to 10% (w/w) water. In some embodiments, powder contains ≤ 10% (w/w) water, ≤ 9.5% (w/w) water, ≤ 9% (w/w) water, ≤ 8.5% (w/w) water, ≤ 8% (w/w) water, ≤ 7.5% (w/w) water, ≤ 7% (w/w) water, ≤ 6.5% (w/w) water, ≤ 6% (w/w) water, ≤ 5.5% (w/w) water, ≤ 5% (w/w) water, ≤ 4.5% (w/w) water, ≤ 4% (w/w) water, ≤ 3.5% (w/w) water, ≤ 3% (w/w) water, ≤ 2.5% (w/w) water, ≤ 2% (w/w) water, ≤ 1.5% (w/w) water, ≤ 1% (w/w) water or ≤ 0.5% (w/w) water. In some embodiments, the powder contains about 10% (w/w) water, about 9.5% (w/w) water, about 9% (w/w) water, about 8.5% (w/w) water, about 8% (w/w) water, about 7.5% (w/w) water, about 7% (w/w) water, about 6.5% (w/w) water, about 6% (w/w) water, about 5.5% (w/w) water, about 5% (w/w) water, about 4.5% (w/w) water, about 4% (w/w) water, about 3.5% (w/w) water, about 3% (w/w) water, about 2.5% (w/w) water, about 2% (w/w) water, about 1.5% (w/w) water, about 1% (w/w) water, about 0.5% (w/w) water, or about 0% (w/w) water. In some embodiments, the powder contains 0.01%-10% (w/w/) water, 0.01%-3% (w/w) water, 0.5%-4% (w/w) water, 3%-10% (w/w) of water, 0%-3% (w/w) water, 0.5%-3.5% (w/w) of water, 1%-4% (w/w) of water, 1.5%-4.5% (w/w) of water, 2%-5% (w/w) of water, 2.5%-5.5% (w/w) of water, 3%-6% (w/w) of water, 3.5%-6.5% (w/w) of water, 4%-7% (w/w) of water, 4.5%-7.5% (w/w) of water, 5%-8% (w/w) of water, 5.5%-8.5% (w/w) of water, 6%-9% (w/w) of water, 6.5%-9.5% (w/w) of water or 7%-10% (w/w) of water.

Water content of the microparticles may be determined by any one or more methods known in the art. Those methods include gravimetric methods, including thermogravimetry, gas chromatography, near-infrared spectroscopy, coulometry, and the Karl Fischer method. These methods are reviewed in J. K. Townes, "Moisture content in proteins: its effects and measurement," 705 J. Chromatography A 115-127, 1995, and references cited therein. For example, the Loss on Drying (LOD) method (gravimetric) may be used in which the microparticles are weighed, subjected to heating to drive off water and other volatiles, and then weighed again. The loss of mass is attributed to water (and other volatiles) contained within the starting material. Near-infrared spectroscopy measures reflectance from 1100 nm to 2500 nm through a glass vial (glass surface) containing the protein to determine moisture content without destroying the sample. See United States Pharmacopeia, XXIII Revision, USP Convention, Rockville, MD 1995, pp. 1801-1802; and Savage et. al., "Determination of Adequate Moisture Content for Efficient Dry-Heat Viral Inactivation in Lyophilized Factor VIII by Loss on Drying and by Near Infrared Spectroscopy," 26 Biologicals 119-124, 1998. A preferred method to determine water content is the Karl Fischer method (volumetric or coulometric), which determines the amount of H₂O by measuring the oxidation of SO₂ by I₂, wherein one mole of I₂ is consumed per mole of H₂O.

Water can stabilize or otherwise contribute to the stability of the protein of the subject formulated pharmaceutical powder, and in some embodiments replace the thermal stabilizer.

In one embodiment, the glycoprotein provided in the formulated pharmaceutical powder is stable. The term "stable" or "stability" refers to the retention of an acceptable degree of physical and chemical structure or biological function of the glycoprotein after storage under defined conditions, or after deposition into a physiologically relevant environment. The protein may be stable even though it does not maintain 100% of its chemical structure or biological function after storage or deposition for a defined amount of time. Under certain circumstances, maintenance of about 80%, about 85%, about 90%, about 95%, about 96%, about 97%, about 98% or about 99% of the protein's structure or function after storage or deposition for a defined amount of time may be regarded as "stable".

Stability can be measured *inter alia* by determining the percentage of native molecule that remains in the formulation after storage or deposit into a patient for a defined amount of time at a defined temperature. The percentage of protein that retains its native conformation can be determined by inter alia size exclusion chromatography *(e.g.,* size exclusion high performance liquid chromatography [SE-HPLC]). To determine the stability of the protein, in one embodiment the subject formulated pharmaceutical powder is solubilized and then the protein is subjected to testing. Native protein includes protein that is non-aggregated and non-degraded. In some embodiments, at least about 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100% of the native form of the protein can be detected in the provided formulated pharmaceutical powder after storage for a defined amount of time at a defined temperature or under physiological conditions after deposit within the patient *(e.g.,* implantation).

Aggregated protein can be detected as species migrating as a high molecular weight species on a gel or chromatographic sieve. The term "high molecular weight" or "HMW" species or protein can be used interchangeably with "aggregate," "aggregates," or "aggregated protein." A stable protein of the subject formulated pharmaceutical powder undergoes an increase in the rate of HMW species formation (a.k.a. aggregation rate) of < 10% per month^½, < 9% per month^½, < 8% per month^½, < 7% per month^½, < 6% per month^½, < 5% per month^½, < 4% per month^½, < 3% per month^½, < 2% per month^½or < 1% per month^½. In a preferred embodiment, the aggregation rate of the protein provided in the formulated pharmaceutical powder is < 5% per month^½.

The defined amount of time after which stability is measured can be at least 14 days, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, or more. The temperature at which the microparticles may be kept when assessing stability can be any temperature from about -80°C to about 50°C, *e.g.,* storage at about -80°C, about -30°C, about - 20°C, about 0°C, about 4°-8°C, about 5°C, about 25°C or other ambient temperatures, about 35°C, about 37°C or other physiological temperatures, about 45°C or about 50°C.

Stability can be measured by determining the percentage of protein that forms an aggregate (*i.e*., high molecular weight species) after a defined amount of time at a defined temperature, wherein stability is inversely proportional to the percent high molecular weight (HMW) species that is formed. The percentage of HMW species of the protein can be determined by size exclusion chromatography after solubilization, as described above. A protein microparticle may also be deemed stable if after three months at ambient temperature less than about 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, 0.5%, or 0.1% of the protein is detected in a HMW form. Preferably, < 10%, < 5% or < 2% of the protein provided in the formulated pharmaceutical powder is present as a HMW species.

Stability can be measured by determining the percentage of protein that is degraded or otherwise is found as a low molecular weight (LMW) species within the microparticle after a defined amount of time at a defined temperature, wherein stability is inversely proportional to the percent LMW species that is detected in the solubilized microparticle. The percentage of LMW species of the protein can be determined by size exclusion chromatography, as described above. A protein microparticle may also be deemed stable if after three months at ambient temperature less than about 25%, 24%, 23%, 22%, 21%, 20%, 19%, 18%, 17%, 16%, 15%, 14%, 13%, 12%, 11%, 10%, 9%, 8%, 7%, 6%, 5%, 4%, 3%, 2%, 1%, 0.5%, or 0.1% of the first molecule is detected in a LMW form.

In some embodiments, the subject formulated pharmaceutical powder contains a buffer. Buffers are well-known in the art. In general, a buffer is included in the aqueous protein solution feedstock used to prepare the subject powder. In some embodiments the buffer is included in the feedstock at a concentration of 1 mM to 100 mM. In some particular embodiments, the buffer is included in the feedstock at about 10 mM. In certain embodiments, the buffer is present in the feedstock at a concentration of 5 mM ± 0.75 mM to 15 mM ± 2.25 mM; 6 mM ± 0.9 mM to 14 mM ± 2.1 mM; 7 mM ± 1.05 mMto 13 mM ± 1.95 mM; 8 mM ± 1.2 mMto 12 mM ± 1.8 mM; 9 mM ± 1.35 mM to 11 mM ± 1.65 mM; 10 mM ± 1.5 mM; or about 10 mM. In some embodiments, the buffer system of the feedstock comprises histidine, phosphate, and/or acetate at 10 mM ± 1.5 mM.

In some embodiments, the buffer is present in the subject formulated pharmaceutical powder at a concentration of ≤ 10% (w/w), ≤ 9.5% (w/w), ≤ 9% (w/w), ≤ 8.5% (w/w), ≤ 8% (w/w), ≤ 7.5% (w/w), ≤ 7% (w/w), ≤ 6.5% (w/w), ≤ 6% (w/w), ≤ 5.5% (w/w), ≤ 5% (w/w), ≤ 4.5% (w/w), ≤ 4% (w/w), ≤ 3.5% (w/w), ≤ 3% (w/w), ≤ 2.5% (w/w), ≤ 2% (w/w), ≤ 1.5% (w/w), ≤ 1% (w/w) or ≤ 0.5% (w/w). In some embodiments, the buffer is present in the subject formulated pharmaceutical powder at a concentration of 0.1-0.5% (w/w), 0.5-1% (w/w), 0.5-1.5% (w/w), 1-2% (w/w), 1.5-2.5% (w/w), 2-3% (w/w), 2.5-3.5% (w/w), 3-4% (w/w), 3.5-4.5% (w/w), 4-5% (w/w), 4.5-5.5% (w/w), 5-6% (w/w), 5.5-6.5% (w/w), 6-7% (w/w), 6.5-7.5% (w/w), 8-9% (w/w), 8.5-9.5% (w/w) or 9-10% (w/w).

In some embodiments, the buffer is selected from buffers that are inclusively encompassed within the pH range of about 3 to about 9, or within the pH range of about 3.7 to about 8.0. For example, the protein-containing aqueous solution feedstock may have a pH of about 3.4, about 3.6, about 3.8, about 4.0, about 4.2, about 4.4, about 4.6, about 4.8, about 5.0, about 5.2, about 5.4, about 5.6, about 5.8, about 6.0, about 6.2, about 6.4, about 6.6, about 6.8, about 7.0, about 7.2, about 7.4, about 7.6, about 7.8, or about 8.0.

The buffer may be a combination of individual buffers, such as *e.g.,* the combination of histidine and acetate (his-acetate buffer). In one embodiment, the buffer has a buffering range of about 3.5 to about 6, or about 3.7 to about 5.6, such as the range buffered by acetate. In one embodiment, the buffer has a buffering range of about 5.5 to about 8.5, or about 5.8 to about 8.0, such as the range buffered by phosphate. In one embodiment, the buffer has a buffering range of about 5.0 to about 8.0, or about 5.5 to about 7.4, such as the range buffered by histidine.

In one embodiment, the subject formulated pharmaceutical contains no additional buffer. Here, any buffering capacity of the powder or liquid aqueous feed-stock is provided by the included protein, thermal stabilizer if present, or the water.

A surfactant (one or more) may also be included in the pre-microparticle protein-containing aqueous feedstock. As used herein, the term "surfactant" means a substance which reduces the surface tension of a fluid in which it is dissolved and/or reduces the interfacial tension between oil and water. Surfactants can be ionic or non-ionic. Exemplary non-ionic surfactants that can be included in the feedstock (and subsequently the formulated pharmaceutical powder) include, e.g., alkyl poly(ethylene oxide), alkyl polyglucosides (e.g., octyl glucoside and decyl maltoside), fatty alcohols such as cetyl alcohol and oleyl alcohol, cocamide MEA, cocamide DEA, and cocamide TEA. Specific non-ionic surfactants that can be included in the feedstock include, e.g., polyoxyethylene sorbitan esters (a.k.a. polysorbates) such as polysorbate 20, polysorbate 28, polysorbate 40, polysorbate 60, polysorbate 65, polysorbate 80, polysorbate 81, and polysorbate 85; poloxamers such as poloxamer 188, poloxamer 407; polyethylene-polypropylene glycol; or polyethylene glycol (PEG). Polysorbate 20 is also known as TWEEN 20, sorbitan monolaurate and polyoxyethylenesorbitan monolaurate. Claimed are nonionic surfactants selected from the group consisting of alkyl polyethylene oxide, alkyl polyglucosides, fatty alcohols, cocamide MEA, cocamide DEA, cocamide TEA, polyoxyethylene sorbitan esters, poloxamers, poloxamer 188, poloxamer 407, polyethylene-polypropylene glycol, and polyethylene glycol.

The amount of surfactant contained within the feedstock solution may vary depending on the specific properties and purposes desired of the powder. The bulk properties of the powder such as flowability can be affected by regulating surfactant content. While not wishing to be bound by theory, the surfactant affects the air-surface interface of aqueous protein droplets (precursor to the subject powder) by reducing surface tension. Higher concentrations of surfactant produce rounder and smoother protein microparticles, which can improve flowability. Lower concentrations of surfactant produce less round and more dimpled protein microparticles.

In certain embodiments, the precursor aqueous solution containing the subject protein may contain about 0.015% (w/v) to about 0.1% (w/v) surfactant (e.g., polysorbate 20 or polysorbate 80). For example, the feedstock may contain about 0.015%; about 0.016%; about 0.017%; about 0.018%; about 0.019%; about 0.02%; about 0.021%; about 0.022%; about 0.023%; about 0.024%; about 0.025%; about 0.026%; about 0.027%; about 0.028%; about 0.029%; about 0.03%; about 0.031%; about 0.032%; about 0.033%; about 0.034%; about 0.035%; about 0.036%; about 0.037%; about 0.038%; about 0.039%; about 0.04%; about 0.041%; about 0.042%; about 0.043%; about 0.044%; about 0.045%; about 0.046%; about 0.047%; about 0.048%; about 0.049%; about 0.05%; about 0.051%; about 0.052%; about 0.053%; about 0.054%; about 0.055%; about 0.056%; about 0.057%; about 0.058%; about 0.059%; about 0.06%; about 0.061%; about 0.062%; about 0.063%; about 0.064%; about 0.065%; about 0.066%; about 0.067%; about 0.068%; about 0.069%; about 0.07%; about 0.071%; about 0.072%; about 0.073%; about 0.074%; about 0.075%; about 0.076%; about 0.077%; about 0.078%; about 0.079%; about 0.08%; about 0.081%; about 0.082%; about 0.083%; about 0.084%; about 0.085%; about 0.086%; about 0.087%; about 0.088%; about 0.089%; about 0.09%; about 0.091%; about 0.092%; about 0.093%; about 0.094%; about 0.095%; about 0.096%; about 0.097%; about 0.098%; about 0.099%; or about 0.10% surfactant (e.g., polysorbate 20 or polysorbate 80).

In one embodiment, the formulated pharmaceutical powder comprises an amphipathic nonionic surfactant, such as a fatty acid ester of a polyoxyethylene sorbitan. In one embodiment, the surfactant is polysorbate 20 or polysorbate 80. In one embodiment, the ratio by weight of polysorbate to protein in the subject formulated pharmaceutical powder is 0.003:1 - 1:5, 0.03:10, 0.3:50, 0.3:25, 1:50, 0.3:10, 3:50, 3:25 or 1:5.

Thermal stabilizers can be included in the formulated pharmaceutical powder (and therefore any precursor aqueous to inhibit or reduce the formation of aggregates and other degradation products during thermal stress. Thermal stabilizers can be amino acids, preferable amino acids with hydrophobic side chains, carbohydrates, sugar alcohols, polymers, copolymers, and block copolymers, polypeptides, surfactants, and the like. Examples of useful thermal stabilizers include pluronic F68, arginine, lysine, those amino acids with hydrophobic side chains including glycine, proline, valine, leucine, and isoleucine, sorbitol, mannitol, glycerol, trehalose, dextrose, sucrose and other carbohydrates, various cyclodextrins, sodium chloride, and combinations thereof. See Goldberg et al., "Formulation development of therapeutic monoclonal antibodies using high-throughput fluorescence and static light scattering techniques: role of conformational and colloidal stability," 100(4) J. Pharm. Sci. 1306-1315, 2011, and Bhambhani et al., "Formulation design and high-throughput excipient selection based on structural integrity and conformational stability of dilute and highly concentrated IgG1 monoclonal antibody solutions," 101(3) J. Pharm. Sci. 1120-1135, 2012. The thermal stabilizers claimed are selected from sucrose, trehalose, mannitol, isoleucine, proline, or combinations thereof.

The carbohydrate can be a reducing sugar or a non-reducing sugar. "Reducing sugars" include, e.g., sugars with a ketone or aldehyde group and contain a reactive hemiacetal group, which allows the sugar to act as a reducing agent. Specific examples of reducing sugars include fructose, glucose, glyceraldehyde, lactose, arabinose, mannose, xylose, ribose, rhamnose, galactose and maltose. Non-reducing sugars can comprise an anomeric carbon that is an acetal and is not substantially reactive with amino acids or polypeptides to initiate a Maillard reaction. Specific examples of non-reducing sugars include sucrose, trehalose, sorbose, sucralose, melezitose and raffinose. Sugar acids include, for example, saccharic acids, gluconate and other polyhydroxy sugars and salts thereof.

In some embodiments, thermal stabilizers are included on a mass-by-mass or mole-by-mole ratio with the protein. While not wishing to be bound by theory, thermal stabilizers are believed in part to replace the water that surrounded the protein to help maintain protein stability (water replacement therapy) thereby allowing for the removal of water while maintaining protein structure. According to an aspect of the invention, the ratio of protein to stabilizer is maximized to permit the formulation of higher amounts of protein per unit volume while maintaining proper protein structure. In one embodiment, for every 5 parts of protein by weight the formulated pharmaceutical powder contains ≤ 2 parts of thermal stabilizer by weight. For example, if the pre-particle feedstock aqueous solution contains 5 mg/ml of protein, then the totality of the thermal stabilizer would be included at ≤ 2 mg/ml to maintain the ratio of 5 parts protein to ≤ 2 parts thermal stabilizer in the subject formulated pharmaceutical powder. In one embodiment, for every 5 parts of protein by weight the formulated pharmaceutical powder contains ≤ 1 part of thermal stabilizer by weight. For example, if the feedstock aqueous solution contains 5 mg/ml of protein, then the totality of the thermal stabilizer would be included at ≤ 1 mg/ml to maintain the ratio of 5 parts protein by weight to 1 part thermal stabilizer by weight in the subject formulated pharmaceutical powder. In some embodiments, the weight to weight ratio of protein to thermal stabilizer is 5:2 - 100:1, 5:2 - 10:3, 20:7 - 4:1, 10:3 - 5:1, 4:1 - 20:3, 5:1 - 10:1, 20:3 - 20:1, 10:1 - 40:1, 20:1 - 50:1, or 40:1 - 100:1.

In another embodiment, for every mole of protein the formulated pharmaceutical powder contains < 300 moles of thermal stabilizer. For example, if the precursor aqueous solution feedstock contains 1 mM of protein, then the totality of the thermal stabilizer would be included at < 300 mM to maintain the molar ratio of thermal stabilizer to protein of < 300:1 in the subject formulated pharmaceutical powder. In some embodiments, the molar ratio of thermal stabilizer to protein is 350:1 - 1:1, 350:1 - 300:1, 325:1 - 275:1, 300:1 - 250:1, 275:1 - 225:1, 250:1-200:1, 225:1 - 175:1, 200:1 - 150:1, 175:1 - 125:1, 150:1 - 100:1, 125:1 - 75:1, 100:1 - 50:1, 75:1 - 25:1, or 50:1 - ≤ 1:1.

The thermal stabilizer component may contain more than one molecular species. For example, the thermal stabilizer may consist of any one or more of sucrose, trehalose, mannitol, arginine (Arg) and the hydrophobic amino acids glycine (Gly), alanine (Ala), valine (Val), leucine (Leu), isoleucine (Ile), proline (Pro), phenylalanine (Phe), methionine (Met), and tryptophan (Trp) in various relative amounts adding up to the total cumulative quantity of thermal stabilizer. Tables 1 and 2 provide examples of useful ratios of thermal stabilizer and protein in specific aqueous feedstock solutions, and Table 4 provides examples of useful ratios of thermal stabilizer and protein in specific formulated pharmaceutical powders and the stability of the protein in powder derived from those feedstock formulations of Table 3. The thermal stabilizers claimed are selected from sucrose, trehalose, mannitol, isoleucine, proline, or combinations thereof.

In some embodiments, the subject thermal stabilizer can be a large molecule (> 200 grams per mole). Large molecules that are useful as a thermal stabilizer include disaccharides such as sucrose, trehalose, lactose, maltose, cellobiose and the like. Sucrose and trehalose are preferred large molecule stabilizers. The thermal stabilizers claimed are selected from sucrose, trehalose, mannitol, isoleucine, proline, or combinations thereof.

**Table 1: Pharmaceutical Powder Feedstock with Thermal Stabilizers**

| Exemplar | Protein (mg/ml) | Thermal Stabilizer (mg/ml) | | | |
|---|---|---|---|---|---|
| | | Sucrose | Trehalose | Mannitol | Isoleucine |
| 1 | 50 | 10 | - | - | - |
| 2 | 50 | 20 | - | - | - |
| 3 | 50 | 10 | - | 5 | 5 |
| 4 | 50 | - | 10 | - | - |
| 5 | 50 | - | 20 | - | - |
| 6 | 50 | - | 10 | 5 | 5 |
| 7 | 50 | - | - | 10 | 10 |
| 8 | 50 | - | - | - | - |
| 9 | 50 | - | 10 | - | - |
| 10 | 50 | - | 20 | - | - |
| 11 | 50 | - | 10 | - | - |
| 12 | 5 | 1 | - | - | - |
| 13 | 5 | 2 | - | - | - |
| 14 | 5 | 1 | - | 0.5 | 0.5 |
| 15 | 5 | - | 1 | - | - |
| 16 | 5 | - | 2 | - | - |
| 17 | 5 | - | 1 | 0.5 | 0.5 |
| 18 | 5 | - | - | 1 | 1 |

**Table 2: Pharmaceutical Powder Feedstock with Thermal Stabilizers, Buffer and Surfactant**

| Formulation | Protein (mg/mL) | Phosphate (mM) | Thermal Stabilizer | | | | Polysorbate 20 (% w/v) |
|---|---|---|---|---|---|---|---|
| | | | Sucrose (% w/v) | Trehalose (% w/v) | Mannitol (% w/v) | Isoleucine (% w/v) | |
| 19 | 50 | 10 | 2 | | | | 0.015 |
| 20 | 50 | 10 | 1 | | | | 0.015 |
| 21 | 50 | 10 | 1 | | 0.5 | 0.5 | 0.015 |
| 22 | 50 | 10 | | 2 | | | 0.015 |
| 23 | 50 | 10 | | 1 | 0.5 | 0.5 | 0.015 |
| 24 | 50 | 10 | | | 1 | 1 | 0.015 |
| 25 | 50 | 10 | 2 | | | | 0.03 |
| 26 | 50 | 10 | 1 | | | | 0.03 |
| 27 | 50 | 10 | 1 | | 0.5 | 0.5 | 0.03 |
| 28 | 50 | 10 | | 2 | | | 0.03 |
| 29 | 50 | 10 | | 1 | 0.5 | 0.5 | 0.03 |
| 30 | 50 | 10 | | | 1 | 1 | 0.03 |
| 31 | 50 | 10 | 2 | | | | 0.06 |
| 32 | 50 | 10 | 1 | | | | 0.06 |
| 33 | 50 | 10 | 1 | | 0.5 | 0.5 | 0.06 |
| 34 | 50 | 10 | | 2 | | | 0.06 |
| 35 | 50 | 10 | | 1 | 0.5 | 0.5 | 0.06 |
| 36 | 50 | 10 | | | 1 | 1 | 0.06 |
| 37 | 50 | 10 | 2 | | | | 0.1 |
| 38 | 50 | 10 | 1 | | | | 0.1 |
| 39 | 50 | 10 | 1 | | 0.5 | 0.5 | 0.1 |
| 40 | 50 | 10 | | 2 | | | 0.1 |
| 41 | 50 | 10 | | 1 | 0.5 | 0.5 | 0.1 |
| 42 | 50 | 10 | | | 1 | 1 | 0.1 |
| 43 | 5 | 1 | 0.2 | | | | 0.015 |
| 44 | 5 | 1 | 0.1 | | | | 0.015 |
| 45 | 5 | 1 | 0.1 | | 0.05 | 0.05 | 0.015 |
| 46 | 5 | 1 | | 0.2 | | | 0.015 |
| 47 | 5 | 1 | | 0.1 | 0.05 | 0.05 | 0.015 |
| 48 | 5 | 1 | | | 0.1 | 0.1 | 0.015 |
| 49 | 5 | 1 | 0.2 | | | | 0.03 |
| 50 | 5 | 1 | 0.1 | | | | 0.03 |
| 51 | 5 | 1 | 0.1 | | 0.05 | 0.05 | 0.03 |
| 52 | 5 | 1 | | 0.2 | | | 0.03 |
| 53 | 5 | 1 | | 0.1 | 0.05 | 0.05 | 0.03 |
| 54 | 5 | 1 | | | 0.1 | 0.1 | 0.03 |
| 55 | 5 | 1 | 0.2 | | | | 0.06 |
| 56 | 5 | 1 | 0.1 | | | | 0.06 |
| 57 | 5 | 1 | 0.1 | | 0.05 | 0.05 | 0.06 |
| 58 | 5 | 1 | | 0.2 | | | 0.06 |
| 59 | 5 | 1 | | 0.1 | 0.05 | 0.05 | 0.06 |
| 60 | 5 | 1 | | | 0.1 | 0.1 | 0.06 |
| 61 | 5 | 1 | 0.2 | | | | 0.1 |
| 62 | 5 | 1 | 0.1 | | | | 0.1 |
| 63 | 5 | 1 | 0.1 | | 0.05 | 0.05 | 0.1 |
| 64 | 5 | 1 | | 0.2 | | | 0. 1 |
| 65 | 5 | 1 | | 0.1 | 0.05 | 0.05 | 0.1 |
| 66 | 5 | 1 | | | 0.1 | 0.1 | 0.1 |

**Table 3: Pharmaceutical Powder Feedstock with Thermal Stabilizers, Buffer and Surfactant**

| Exemplar | Liquid Feedstock Components (mg/mL) | | | | | | | |
|---|---|---|---|---|---|---|---|---|
| | Protein | Sucrose | Trehalose | Mannitol | Isoleucine | Proline | Phosphate | Polysorbate |
| 67 | 50 | 0 | 0 | 0 | 0 | 0 | 1.5 | 0 |
| 68 | 5 | 0.5 | 0.25 | 0 | 0.25 | 0 | 0.15 | 0.15 |
| 69 | 5 | 0.5 | 0 | 0.25 | 0.25 | 0 | 0.15 | 0.15 |
| 70 | 5 | 0 | 0.5 | 0.25 | 0.25 | 0 | 0.15 | 0.15 |
| 71 | 5 | 0.5 | 0 | 0 | 0.5 | 0 | 0.15 | 0.15 |
| 72 | 5 | 0.5 | 0 | 0 | 0 | 0.5 | 0.15 | 0.15 |
| 73 | 5 | 0.5 | 0 | 0.5 | 0 | 0 | 0.15 | 0.15 |
| 74 | 5 | 1 | 0 | 0 | 0 | 0 | 0.15 | 0.15 |
| 75 | 5 | 2 | 0 | 0 | 0 | 0 | 0.15 | 0.2 |
| 76 | 5 | 2 | 0 | 0 | 0 | 0 | 0.15 | 0 |
| 77 | 5 | 0 | 2 | 0 | 0 | 0 | 0.15 | 0 |
| 78 | 5 | 0 | 1 | 0.5 | 0.5 | 0 | 0.15 | 0 |
| 79 | 50 | 5 | 0 | 0 | 0 | 0 | 1.5 | 0 |
| 80 | 50 | 10 | 0 | 0 | 0 | 0 | 1.5 | 0 |
| 81 | 50 | 5 | 0 | 2.5 | 2.5 | 0 | 1.5 | 0 |
| 82 | 50 | 20 | 0 | 0 | 0 | 0 | 1.5 | 0 |
| 83 | 50.6 | 9 | 0 | 4.5 | 4.5 | 0 | 1.35 | 0 |
| 84 | 50 | 10 | 0 | 10 | 0 | 0 | 1.5 | 0 |
| 85 | 50 | 10 | 0 | 0 | 10 | 0 | 1.5 | 0 |
| 86 | 50 | 10 | 0 | 5 | 5 | 0 | 1.5 | 0 |
| 87 | 50 | 0 | 0 | 10 | 10 | 0 | 1.5 | 0 |
| 88 | 50 | 0 | 0 | 20 | 0 | 0 | 1.5 | 0 |
| 89 | 50 | 0 | 0 | 0 | 20 | 0 | 1.5 | 0 |

**Table 4: Pharmaceutical Powder Component Ratios and Aggregation Rate**

| Exemplar | % w/w glycoprotein in powder | Thermal stabilizer/glycoprotein weight ratio | Thermal stabilizer/glycoprotein molar ratio | Aggregation rate at 50°C (%HMW/month^{-½}) |
|---|---|---|---|---|
| 67 | 97.5 | 0.0 | 0 | 20.0 |
| 68 | 82.0 | 0.2 | 93 | 10.3 |
| 69 | 82.0 | 0.2 | 109 | 9.4 |
| 70 | 82.0 | 0.2 | 106 | 9.5 |
| 71 | 82.0 | 0.2 | 121 | 8.6 |
| 72 | 82.0 | 0.2 | 133 | 8.4 |
| 73 | 82.0 | 0.2 | 97 | 9.3 |
| 74 | 82.0 | 0.2 | 67 | 11.8 |
| 75 | 71.6 | 0.4 | 134 | 7.6 |
| 76 | 73.4 | 0.4 | 134 | 8.2 |
| 77 | 73.4 | 0.4 | 122 | 8.5 |
| 78 | 73.4 | 0.4 | 212 | 5.7 |
| 79 | 90.1 | 0.1 | 34 | 14.8 |
| 80 | 83.8 | 0.2 | 67 | 12.5 |
| 81 | 83.8 | 0.2 | 109 | 12.2 |
| 82 | 73.4 | 0.4 | 134 | 8.2 |
| 83 | 75.6 | 0.4 | 194 | 7.5 |
| 84 | 73.4 | 0.4 | 193 | 6.2 |
| 85 | 73.4 | 0.4 | 243 | 6.0 |
| 86 | 73.4 | 0.4 | 218 | 5.4 |
| 87 | 73.4 | 0.4 | 302 | 4.9 |
| 88 | 73.4 | 0.4 | 253 | 6.8 |
| 89 | 73.4 | 0.4 | 351 | 7.8 |

In some embodiments, the subject thermal stabilizer comprises one or more small molecules (≤ 200 grams per mole) without a large molecule stabilizer. For a given weight ratio, the inclusion of low molecular weight thermal stabilizers can maximize the thermal stabilizer to protein ratio on a molar basis, thereby providing a benefit to stability. Small molecules that are useful as a thermal stabilizer include monosaccharides such as ribose, deoxyribose, glucose, fructose, galatose, and the like, sugar alcohols and other polyols such as erythritol, glycerol, threitol, arabitol, xylitol, ribitol, mannitol, sorbitol, galactitol, fucitol, iditol, inositol and the like, and amino acids such as arginine and the hydrophobic amino acids glycine, alanine, valine, leucine, isoleucine, proline, phenylalanine, methionine, and tryptophan and the like. The hydrophobic amino acids have aliphatic side chains with very small dipole moments and a carboxylic acid (polar) head. Free hydrophobic amino acids are therefore amphipathic. While not wishing to be bound by theory, the amphipathic thermal stabilizer may provide some protection to the protein against air-liquid interface denaturation. Preferred small molecule thermal stabilizers include mannitol, isoleucine, and proline. The thermal stabilizers claimed are selected from sucrose, trehalose, mannitol, isoleucine, proline, or combinations thereof.

In some embodiments, the thermal stabilizer comprises sucrose only, trehalose only, isoleucine only, mannitol only or proline only. In some embodiments, the thermal stabilizer comprises a combination of sucrose and trehalose. In some embodiments, the thermal stabilizer comprises a combination of sucrose and trehalose in a by weight ratio of 3:1 - 1:3, about 2:1, about 4:3, about 1:1, about 3:4, or about 1:2.

In some embodiments, the thermal stabilizer comprises a combination of sucrose, mannitol and isoleucine. In some embodiments, the thermal stabilizer comprises a combination of sucrose, mannitol and isoleucine in a by weight ratio of 4:3:1, 2:1:1, 4:1:3, 2:3:1, 1:1:1, or 2:1:3.

In some embodiments, formulated pharmaceutical powders with sucrose only have higher aggregation rates than those using mannitol, isoleucine, proline, or combinations thereof (Figure 6).

In some embodiments, the thermal stabilizer comprises a combination of trehalose, mannitol and isoleucine. In some embodiments, the thermal stabilizer comprises a combination of trehalose, mannitol and isoleucine in a by weight ratio of 4:3:1, 2:1:1, 4:1:3, 2:3:1, 1:1:1, or 2:1:3.

In some embodiments, the thermal stabilizer comprises a combination of sucrose and isoleucine. In some embodiments, the thermal stabilizer comprises a combination of sucrose and trehalose in a by weight ratio of 3:1 - 1:3, about 2:1, about 4:3, about 1:1, about 3:4, or about 1:2.

In some embodiments, the thermal stabilizer comprises a combination of sucrose and proline. In some embodiments, the thermal stabilizer comprises a combination of sucrose and proline in a by weight ratio of 3:1 - 1:3, about 2:1, about 4:3, about 1:1, about 3:4, or about 1:2.

In some embodiments, the thermal stabilizer comprises a combination of sucrose and mannitol. In some embodiments, the thermal stabilizer comprises a combination of sucrose and mannitol in a by weight ratio of 3:1 - 1:3, about 2:1, about 4:3, about 1:1, about 3:4, or about 1:2.

In some embodiments, the thermal stabilizer comprises a combination of mannitol and isoleucine. In some embodiments, the thermal stabilizer comprises a combination of mannitol and isoleucine in a by weight ratio of 3:1 - 1:3, about 2:1, about 4:3, about 1:1, about 3:4, or about 1:2.

In one embodiment, the thermal stabilizer is sucrose present in a by weight ratio to protein in the provided formulated pharmaceutical powder of about 1:5 to 2:5. In another embodiment, the formulated pharmaceutical powder comprises sucrose, mannitol, isoleucine and protein in a by weight ratio of about 2:1:1:10.

In one embodiment, the thermal stabilizer is trehalose present in a by weight ratio to protein in the provided formulated pharmaceutical powder of about 1:5 to 2:5. In another embodiment, the formulated pharmaceutical powder comprises trehalose, mannitol, isoleucine and protein in a by weight ratio of about 2:1:1:10.

In one embodiment, the formulated pharmaceutical powder comprises about 71-75% (w/w) glycoprotein, 14-15% (w/w) mannitol, 14-15% (w/w) isoleucine or proline, 2-2.5% (w/w) buffer, and 3-8% (w/w) water.

In some embodiments, the formulated pharmaceutical powder is produced from an aqueous solution containing the subject protein and other excipients. This precursor aqueous solution is also referred to as a "feedstock". In some embodiments, the feedstock may contain on a weight-to-volume (w/v) basis about 0.005% to about 5% thermal stabilizer; about 0.01% to about 4% thermal stabilizer; about .02% to about 3% thermal stabilizer; or about 0.05% to about 2% thermal stabilizer. In some embodiments, the formulated pharmaceutical powder of the present invention may comprise (w/v) about 0.01%; 0.02%; 0.03%; 0.04%; 0.05%; 0.06%; 0.07%; 0.08%; 0.09%; 0.10%; 0.11%; 0.12%; 0.13%; 0.14%; 0.15%; 0.16%; 0.17%; 0.18%; 0.19%; 0.20%; 0.21%; 0.22%; 0.23%; 0.24%; 0.25%; 0.26%; 0.27%; 0.28%; 0.29%; 0.30%; 0.31%; 0.32%; 0.33%; 0.34%; 0.35%; 0.36%; 0.37%; 0.38%; 0.39%; 0.40%; 0.41%; 0.42%; 0.43%; 0.44%; 0.45%; 0.46%; 0.47%; 0.48%; 0.49%; 0.50%; 0.51%; 0.52%; 0.53%; 0.54%; 0.55%; 0.56%; 0.57%; 0.58%; 0.59%; 0.60%; 0.61%; 0.62%; 0.63%; 0.64%; 0.65%; 0.66%; 0.67%; 0.68%; 0.69%; 0.70%; 0.71%; 0.72%; 0.73%; 0.74%; 0.75%; 0.76%; 0.77%; 0.78%; 0.79%; 0.80%; 0.81%; 0.82%; 0.83%; 0.84%; 0.85%; 0.86%; 0.87%; 0.88%; 0.89%; 0.90%; 0.91%; 0.92%; 0.93%; 0.94%; 0.95%; 0.96%; 0.97%; 0.98%; 0.99%; 1.0%; 1.1%; 1.2%; 1.3%; 1.4%; 1.5%; 1.6%; 1.7%; 1.8%; 1.9%; 2.0%; 2.1%; 2.2%; 2.3%; 2.4%; 2.5%; 2.6%; 2.7%; 2.8%; 2.9%; 3.0%; 3.1%; 3.2%; 3.3%; 3.4%; 3.5%; 3.6%; 3.7%; 3.8%; 3.9%; 4.0%; 4.1%; 4.2%; 4.3%; 4.4%; 4.5%; 4.6%; 4.7%; 4.8%; 4.9%; or about 5.0% thermal stabilizers.

In an aspect of the invention where no thermal stabilizer is present in the powder, residual water serves to stabilize the protein.

The protein provided in the subject formulated pharmaceutical powder is not limited to any particular protein entity. As used herein, "protein" includes therapeutic proteins, recombinant proteins used in research or therapy, trap proteins and other receptor Fc-fusion proteins, chimeric proteins, antibodies, monoclonal antibodies, human antibodies, bispecific antibodies, antibody fragments, nanobodies, recombinant antibody chimeras, cytokines, chemokines, peptide hormones, and the like. Proteins may be produced using recombinant cell-based production systems, such as the insect bacculovirus system, yeast systems (e.g., Pichia sp.), mammalian systems (e.g., CHO cells and CHO derivatives like CHO-K1 cells). Ghaderi et al., "Production platforms for biotherapeutic glycoproteins: Occurrence, impact, and challenges of non-human sialylation," 28 Biotechnol Genet Eng Rev. 147-75 (2012) is mentioned herein by reference for therapeutic proteins and their production. Claimed is that the protein is (a) a Fc containing glycoprotein selected from an antibody or a receptor Fc fusion protein, or (b) a soluble receptor.

In some embodiments, the protein is a therapeutic protein. Therapeutic proteins can be antigen-binding proteins, such as *e.g.,* soluble receptor fragments, antibodies (including IgGs) and derivatives or fragments of antibodies, other Fc containing proteins, including Fc fusion proteins, and receptor-Fc fusion proteins, including the trap-type proteins such as *e.g.* aflibercept (a VEGF-trap molecule), rilonacept (an II,1-trap molecule), and etanercept (a TNF-trap molecule). Huang, C., Curr. Opin. Biotechnol. 20: 692-99 (2009) is mentioned herein for trap-type molecules. U.S. Patents No. 7,303,746 B2, 7,303,747 B2, and 7,374,758 B2 are mentioned herein for aflibercept. U.S. Pat. No. 6,927,044 B2 is mentioned herein for rilonacept. U.S. Pat. No. 8,063,182 B2 is mentioned herein for etanercept.

The term "protein" includes any amino acid polymer having more than about 50 amino acids covalently linked via amide bonds. Proteins contain one or more amino acid polymer chains, generally known in the art as "polypeptides". A protein may contain one or multiple polypeptides to form a single functioning biomolecule. "Polypeptides" generally contain over 50 amino acids, whereas "peptides" generally contain 50 amino acids or less.

Proteins may contain various covalent and non-covalent modifications. Disulfide bridges (i.e., between cysteine residues to form cystine) may be present in some proteins. These covalent links may be within a single polypeptide chain, or between two individual polypeptide chains. For example, disulfide bridges are essential to proper structure and function of insulin, immunoglobulins, protamine, and the like. For a recent review of disulfide bond formation, see Oka and Bulleid, "Forming disulfides in the endoplasmic reticulum," 1833(11) Biochim Biophys Acta 2425-9 (2013).

In addition to disulfide bond formation, proteins may be subject to other post-translational modifications. Those modifications include lipidation *(e.g.,* myristoylation, palmitoylation, farnesoylation, geranylgeranylation, and glycosylphosphatidylinositol (GPI) anchor formation), alkylation (e.g., methylation), acylation, amidation, glycosylation (e.g., addition of glycosyl groups at arginine, asparagine, cysteine, hydroxylysine, serine, threonine, tyrosine, and/or tryptophan), and phosphorylation (i.e., the addition of a phosphate group to serine, threonine, tyrosine, and/or histidine). For a recent review on the post-translational modification of proteins produced in eukaryotes, see Mowen and David, "Unconventional post-translational modifications in immunological signaling," 15(6) Nat Immunol 512-20 (2014); and Blixt and Westerlind, "Arraying the post-translational glycoproteome (PTG)," 18 Curr Opin Chem Biol. 62-9 (2014).

In one embodiment, the protein provided in the formulated pharmaceutical powder is a glycoprotein, which encompasses any protein with a glycosyl group. Antibodies and receptor-Fc-fusion proteins (a.k.a. trap proteins or trap molecules) are examples of glycoproteins. Antibodies produced in heterologous mammalian systems are also glycosylated at various residues (e.g., at asparagine residues) with various polysaccharides, and can differ from species to species, which may affect antigenicity for therapeutic antibodies (see Butler and Spearman, "The choice of mammalian cell host and possibilities for glycosylation engineering", 30 Curr Opin Biotech 107-112 (2014)). The N-glycans of IgG molecules generally include mannosylated N-acetylglucosamine, which in some cases may include additional galactose and/or fucose groups. Antibodies generally have a mass of about 150 - 170 kD (kg/mol) with glycosylation providing about 2-3% of the mass. See Plomp et al., "Recent Advances in Clinical Glycoproteomics of Immunoglobulins (Igs)," Mol Cell Proteomics. 2016 Jul; 15(7):2217-28.

Trap molecules are similarly N-glycosylated since they contain an immunoglobulin Fc domain. Trap molecules have a greater range of molecular weight, from about 50 kD for etancept, about 100 kD for aflibercept, to about 250 kD for rilonacept. Given the smaller size of the aflibercept polypeptide chain, the relative level of contribution of glycosylation to the mass of the aflibercept molecule is greater than that of an antibody, *i.e.,* about 5% - 16% of the total mass or more. While not wishing to be bound by theory, the difference in relative glycosylation of different proteins may affect the optimum ratio of thermal stabilizer to protein in the subject formulated pharmaceutical powder.

For example, in one specific embodiment in which the protein is aflibercept, the subject formulated pharmaceutical particle comprises by weight about 62% polypeptide, about 12% glycan, about 25% sucrose, and about 2% phosphate. In another specific embodiment in which the protein is aflibercept, the subject formulated pharmaceutical particle comprises by weight about 71% polypeptide, about 13% glycan, about, 2% phosphate, and about 14.1% sucrose.

For example, in a specific embodiment in which the protein is an IgG1 molecule, the subject formulated pharmaceutical particle comprises by weight about 81% protein, about 1.5% histidine, about 16% sucrose, and about 0.2% polysorbate 80.

For example, in a specific embodiment in which the protein is an IgG4 molecule, the subject formulated pharmaceutical particle comprises by weight about 45% protein, about 0.4% acetate, about 56% sucrose, and about 0.4% polysorbate 20s.

Immunoglobulins (a.k.a. "antibodies") are examples of proteins having multiple polypeptide chains and extensive post-translational modifications. The canonical immunoglobulin protein (e.g., IgG) comprises four polypeptide chains - two light chains and two heavy chains. Each light chain is linked to one heavy chain via a cystine disulfide bond, and the two heavy chains are bound to each other via two cystine disulfide bonds. Immunoglobulins produced in mammalian systems are also glycosylated at various residues (e.g., at asparagine residues) with various polysaccharides, and can differ from species to species, which may affect antigenicity for therapeutic antibodies. Butler and Spearman, "The choice of mammalian cell host and possibilities for glycosylation engineering", 30 Curr Opin Biotech 107-112 (2014) is mentioned herein by reference to heterologous production of glycoproteins in mammalian cell systems.

Antibodies are often used as therapeutic biomolecules. The term "antibody", as used herein, includes immunoglobulin molecules comprised of four polypeptide chains, two heavy (H) chains and two light (L) chains inter-connected by disulfide bonds. Each heavy chain comprises a heavy chain variable region (abbreviated herein as HCVR or VH) and a heavy chain constant region. The heavy chain constant region comprises three domains, CH1, CH2 and CH3. Each light chain comprises a light chain variable region (abbreviated herein as LCVR or VL) and a light chain constant region. The light chain constant region comprises one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4 (heavy chain CDRs may be abbreviated as HCDR1, HCDR2 and HCDR3; light chain CDRs may be abbreviated as LCDR1, LCDR2 and LCDR3. The term "high affinity" antibody refers to those antibodies having a binding affinity to their target of at least 10-9 M, at least 10-1 M; at least 10-11 M; or at least 10-12 M, as measured by surface plasmon resonance, *e.g.,* BIACORE^{™} or solution-affinity ELISA.

The phrase "bispecific antibody" includes an antibody capable of selectively binding two or more epitopes. Bispecific antibodies generally comprise two different heavy chains, with each heavy chain specifically binding a different epitope-either on two different molecules *(e.g.,* antigens) or on the same molecule *(e.g.,* on the same antigen). If a bispecific antibody is capable of selectively binding two different epitopes (a first epitope and a second epitope), the affinity of the first heavy chain for the first epitope will generally be at least one to two or three or four orders of magnitude lower than the affinity of the first heavy chain for the second epitope, and vice versa. The epitopes recognized by the bispecific antibody can be on the same or a different target *(e.g.,* on the same or a different protein). Bispecific antibodies can be made, for example, by combining heavy chains that recognize different epitopes of the same antigen. For example, nucleic acid sequences encoding heavy chain variable sequences that recognize different epitopes of the same antigen can be fused to nucleic acid sequences encoding different heavy chain constant regions, and such sequences can be expressed in a cell that expresses an immunoglobulin light chain. A typical bispecific antibody has two heavy chains each having three heavy chain CDRs, followed by (N-terminal to C-terminal) a CH1 domain, a hinge, a CH2 domain, and a CH3 domain, and an immunoglobulin light chain that either does not confer antigen-binding specificity but that can associate with each heavy chain, or that can associate with each heavy chain and that can bind one or more of the epitopes bound by the heavy chain antigen-binding regions, or that can associate with each heavy chain and enable binding or one or both of the heavy chains to one or both epitopes.

The phrase "heavy chain," or "immunoglobulin heavy chain" includes an immunoglobulin heavy chain constant region sequence from any organism, and unless otherwise specified includes a heavy chain variable domain. Heavy chain variable domains include three heavy chain CDRs and four FR regions, unless otherwise specified. Fragments of heavy chains include CDRs, CDRs and FRs, and combinations thereof. A typical heavy chain has, following the variable domain (from N-terminal to C-terminal), a CH1 domain, a hinge, a CH2 domain, and a CH3 domain. A functional fragment of a heavy chain includes a fragment that is capable of specifically recognizing an antigen *(e.g.,* recognizing the antigen with a KD in the micromolar, nanomolar, or picomolar range), that is capable of expressing and secreting from a cell, and that comprises at least one CDR.

The phrase "light chain" includes an immunoglobulin light chain constant region sequence from any organism, and unless otherwise specified includes human kappa and lambda light chains. Light chain variable (VL) domains typically include three light chain CDRs and four framework (FR) regions, unless otherwise specified. Generally, a full-length light chain includes, from amino terminus to carboxyl terminus, a VL domain that includes FR1-CDR1-FR2-CDR2-FR3-CDR3-FR4, and a light chain constant domain. Light chains that can be used with this invention include those, *e.g.,* that do not selectively bind either the first or second antigen selectively bound by the antigen-binding protein. Suitable light chains include those that can be identified by screening for the most commonly employed light chains in existing antibody libraries (wet libraries or in silico), where the light chains do not substantially interfere with the affinity and/or selectivity of the antigen-binding domains of the antigen-binding proteins. Suitable light chains include those that can bind one or both epitopes that are bound by the antigen-binding regions of the antigen-binding protein.

The phrase "variable domain" includes an amino acid sequence of an immunoglobulin light or heavy chain (modified as desired) that comprises the following amino acid regions, in sequence from N-terminal to C-terminal (unless otherwise indicated): FR1, CDR1, FR2, CDR2, FR3, CDR3, FR4. A "variable domain" includes an amino acid sequence capable of folding into a canonical domain (VH or VL) having a dual beta sheet structure wherein the beta sheets are connected by a disulfide bond between a residue of a first beta sheet and a second beta sheet.

The phrase "complementarity determining region," or the term "CDR," includes an amino acid sequence encoded by a nucleic acid sequence of an organism's immunoglobulin genes that normally (i.e., in a wild-type animal) appears between two framework regions in a variable region of a light or a heavy chain of an immunoglobulin molecule *(e.g.,* an antibody or a T cell receptor). A CDR can be encoded by, for example, a germline sequence or a rearranged or unrearranged sequence, and, for example, by a naive or a mature B cell or a T cell. In some circumstances *(e.g.,* for a CDR3), CDRs can be encoded by two or more sequences *(e.g.,* germline sequences) that are not contiguous *(e.g.,* in an unrearranged nucleic acid sequence) but are contiguous in a B cell nucleic acid sequence, *e.g.,* as the result of splicing or connecting the sequences *(e.g.,* V-D-J recombination to form a heavy chain CDR3).

The phrase "Fc-containing protein" includes antibodies, bispecific antibodies, immunoadhesins, and other binding proteins that comprise at least a functional portion of an immunoglobulin CH2 and CH3 region. A "functional portion" refers to a CH2 and CH3 region that can bind a Fc receptor *(e.g.,* an FcyR; or an FcRn, i.e., a neonatal Fc receptor), and/or that can participate in the activation of complement. If the CH2 and CH3 region contains deletions, substitutions, and/or insertions or other modifications that render it unable to bind any Fc receptor and also unable to activate complement, the CH2 and CH3 region is not functional.

Fc-containing proteins can comprise modifications in immunoglobulin domains, including where the modifications affect one or more effector function of the binding protein *(e.g.,* modifications that affect FcyR binding, FcRn binding and thus half-life, and/or CDC activity). Such modifications include, but are not limited to, the following modifications and combinations thereof, with reference to EU numbering of an immunoglobulin constant region: 238, 239, 248, 249, 250, 252, 254, 255, 256, 258, 265, 267, 268, 269, 270, 272, 276, 278, 280, 283, 285, 286, 289, 290, 292, 293, 294, 295, 296, 297, 298, 301, 303, 305, 307, 308, 309, 311, 312, 315, 318, 320, 322, 324, 326, 327, 328, 329, 330, 331, 332, 333, 334, 335, 337, 338, 339, 340, 342, 344, 356, 358, 359, 360, 361, 362, 373, 375, 376, 378, 380, 382, 383, 384, 386, 388, 389, 398, 414, 416, 419, 428, 430, 433, 434, 435, 437, 438, and 439.

For example, and not by way of limitation, the binding protein is an Fc-containing protein and exhibits enhanced serum half-life (as compared with the same Fc-containing protein without the recited modification(s)) and have a modification at position 250 (e.g., E or Q); 250 and 428 *(e.g.,* L or F); 252 *(e.g.,* L/Y/F/W or T), 254 *(e.g.,* S or T), and 256 *(e.g.,* S/R/Q/E/D or T); or a modification at 428 and/or 433 *(e.g.,* L/R/SI/P/Q or K) and/or 434 *(e.g.,* H/F or Y); or a modification at 250 and/or 428; or a modification at 307 or 308 *(e.g.,* 308F, V308F), and 434. In another example, the modification can comprise a 428L *(e.g.,* M428L) and 434S *(e.g.,* N434S) modification; a 428L, 259I *(e.g.,* V259I), and a 308F *(e.g.,* V308F) modification; a 433K *(e.g.,* H433K) and a 434 *(e.g.,* 434Y) modification; a 252, 254, and 256 *(e.g.,* 252Y, 254T, and 256E) modification; a 250Q and 428L modification *(e.g.,* T250Q and M428L); a 307 and/or 308 modification *(e.g.,* 308F or 308P).

Some recombinant Fc-containing proteins contain receptors or receptor fragments, ligands or ligand fragments that have cognate binding partners in biological systems. "Receptor Fc-fusion proteins" refer to recombinant molecules that contain a soluble receptor fused to an immunoglobulin Fc domain. Some receptor Fc-fusion proteins may contain ligand binding domains of multiple different receptors that affect a given. Those receptor Fc-fusion proteins are known as "traps" or "trap molecules". Rilonocept and aflibercept are examples of marketed traps that antagonize ILIR (see US Pat. No. 7,927,583) and VEGF (see US Pat. No. 7,087,411), respectively. Other recombinant Fc-containing proteins include those recombinant proteins containing a peptide fused to an Fc domain, for example Centocor's MIMETIBODY^{™} technology. Recombinant Fc-containing proteins are described in C. Huang, "Receptor-Fc fusion therapeutics, traps, and MIMETIBODY technology," 20(6) Curr. Opin. Biotechnol. 692-9 (2009).

"Fc-fusion proteins" comprise part or all of two or more proteins, one of which is an Fc portion of an immunoglobulin molecule, that are not fused in their natural state. Preparation of fusion proteins comprising certain heterologous polypeptides fused to various portions of antibody-derived polypeptides (including the Fc domain) has been described, e.g., by Ashkenazi et al., Proc. Natl. Acad. ScL USA 88: 10535, 1991; Byrn et al., Nature 344:677, 1990; and Hollenbaugh et al., "Construction of Immunoglobulin Fusion Proteins", in Current Protocols in Immunology, Suppl. 4, pages 10.19.1 - 10.19.11, 1992. "Receptor Fc fusion proteins" comprise one or more of one or more extracellular domain(s) of a receptor coupled to an Fc moiety, which in some embodiments comprises a hinge region followed by a CH2 and CH3 domain of an immunoglobulin. In some embodiments, the Fc-fusion protein contains two or more distinct receptor chains that bind to a single or more than one ligand(s). For example, an Fc-fusion protein is a trap, such as for example an IL-1 trap *(e.g.,* Rilonacept, which contains the IL-1RAcP ligand binding region fused to the IL-1R1 extracellular region fused to Fc of hIgG1; see U.S. Pat. No. 6,927,004), or a VEGF Trap *(e.g.,* Aflibercept, which contains the Ig domain 2 of the VEGF receptor Flt1 fused to the Ig domain 3 of the VEGF receptor Flk1 fused to Fc of hIgG1; *e.g.,* SEQ ID NO:1; see U.S. Pat. Nos. 7,087,411 and 7,279,159).

"VEGF antagonist" refers to any and all drug, medicament, or other molecule that antagonizes or otherwise reduces the activity of a vascular endothelial growth factor (VEGF). VEGF is a member of the PDGF family of growth factors. It acts predominantly on endothelial cells to promote mitogenesis and cell migration, and thereby stimulate vasculogenesis and angiogenesis. VEGF may be used ectopically to treat ischemia, particularly ischemic heart disease. Conversely, VEGF antagonists may be used to inhibit angiogenesis. Anti-angiogenics are useful in the treatment of cancer, since tumors require neovascularization to continue to grow and to metastasize, and in the treatment of choroidal neovascularization which leads to wet age-related macular degeneration. For a review of VEGF and drugs used to promote or inhibit VEGF activity, see Wu et al., "A systems biology perspective on sVEGFR1: its biological function, pathogenic role and therapeutic use," 14(3) J. Cell Mol. Med. 528-52 (2010); Yadav et al., "Tumour Angiogenesis and Angiogenic Inhibitors: A Review," 9(6) J. Clin. Diagn. Res. XE01-XE05 (2015); and I. Zachary, "VEGF signalling: integration and multi-tasking in endothelial cell biology," 31(Pt 6) Biochem. Soc. Trans. 1171-7 (2003).

Inhibitors of VEGF include small molecules that inhibit VEGF-stimulated tyrosine kinases, including for example lapatinib, sunitinib, sorafenib, axitinib, and paxopanib (Yadav, 2015). Macromolecular inhibitors of VEGF include the monoclonal antibody bevacizumab, the Fab fragment ranibizumab, the trap afibercept, and the PEGylated aptamer pegaptanib (Id).

Other growth factors involved in angiogenesis and serving as therapeutic targets neovascularization include inter alia angiopoietin-2 (Ang2) and platelet-derived growth factor (PDGF). Ang2 is a component in the Angiopoietin-Tie (AT) pathway, and as such is involved in vascular remodeling. The AT pathway, in which angiopoietin-1 binds and agonizes the Tie2 cognate receptor, promotes endothelial cell survival and the building, maturation, and maintenance of blood vessels. Ang2 is also involved in lymphangiogenesis. Ang2 also binds to and modulates Tie2 signaling, and appears to function as a Tie2 antagonist in the presence of Ang1. See Thurston and Daly, "The Complex Role of Angiopoietin-2 in the Angiopoietin-Tie Signaling Pathway," 2(9) Cold Spring Harb. Perspect. Med. a006650 (2012), and Chintharlapalli et al., "Angiopoietin-2: an attractive target for improved antiangiogenic tumor therapy," 73(6) Cancer Res. 1649-57 (2013).

Ang2 is involved in cancer and inflammation. It is upregulated in various carcinomas, cytomas, and sarcomas, as well as in sepsis and inflammatory diseases. Ang2 plays a role in leukocyte recruitment. The blockade of Ang2 results in partial inhibition of the growth of human tumor xenografts. (Thurston, 2012.) Antibodies to Ang2 and other peptide inhibitors that bind to Ang2are under development for the treatment of diseases and conditions caused by or exacerbated by angiogenesis. Anti-Ang2 antibodies are described in, *e.g.,* U.S. Pat. Nos. 6,166,185; 7,521,053; 7,205,275; and U.S. Pat. App. Pub. Nos. 2006/0018909; 2006/0246071; 2006/068953; 2007/0154482; and 2011/0027286.

The PDGF system contains a family of dimeric growth factor ligands and and a receptor tyrosine kinases. It comprises five (5) isoforms of ligand: PDGF-AA, PDGF-BB, PDGF-CC, PDGF-DD, and PDGF-AB; and two (2) receptors: PDGFRA (alpha receptor) and PDGFRB (beta receptor). PDGF is involved in embryonic cell division and tissue remodeling and angiogenesis in later development. Kinase inhibitors, *e.g.,* sorafenib, nilotinib, dasatinib, sunitinib, nintedanib, and imatinib are useful PDGF inhibitors for the treatment of cancers. Several anti-PDGFR antibodies are also under development to antagonize PDGF signaling for the treatment of cancer and other angiogenesis-related diseases. See Kono et al., "Adding to the mix: fibroblast growth factor and platelet-derived growth factor receptor pathways as targets in non-small cell lung cancer," 12(2) Curr. Cancer Drug Targets 107-23 (2012); Bauman et al., "Antagonism of Platelet-Derived Growth Factor Receptor in Non-Small Cell Lung Cancer: Rationale and Investigations," 13 Clin. Cancer Res. 4632s (2007); and Stock et al., "Platelet-derived growth factor receptor-α: a novel therapeutic target in human hepatocellular cancer," 6 Mol. Cancer Ther. 1932-41 (2007); U.S. Pats. No. 5,468,468; 7,740,850; 8,425,911; and 8,574,578; and U.S. Pat. Apps. No. 2009/0053241; 2011/0177074; 2012/0009199; 2012/0027767; and 2014/0193402.

The subject formulated pharmaceutical powder can be manufactured from an aqueous solution (precursor) feedstock. Therefore, the provided protein can be included in the feed stock at certain concentrations. The concentration of protein as well as the total solute concentration in the feedstock may affect the size of the resultant powder-constituent protein microparticle, therefore, the size of the microparticles may be controlled by adjusting the protein concentration or the concentration of other solutes in the feedstock. While not wishing to be bound by theory, lower concentrations of protein or other solutes in the feedstock may produce a finer powder containing smaller constituent protein microparticles. Higher concentrations of protein or other solutes in the feedstock may produce a coarser powder containing larger constituent protein microparticles.

The concentration of protein in the precursor aqueous solution can range from as little as 1 mg/mL or less to as much as practicable, such as about 175-200 mg/mL or more. In some embodiments, the precursor aqueous solution contains the subject protein at concentration of about 1 mg/mL to about 500 mg/mL of protein; about 5 mg/mL to about 400 mg/mL of protein; about 5 mg/mL to about 200 mg/mL of protein; about 25 mg/mL to about 180 mg/mL of protein; about 25 mg/mL to about 150 mg/mL of protein; or about 50 mg/mL to about 180 mg/mL of protein. In some embodiments, the precursor aqueous solution contains the subject protein at concentration of about 1 mg/mL; about 2 mg/mL; about 5 mg/mL; about 10 mg/mL; about 15 mg/mL; about 20 mg/mL; about 25 mg/mL; about 30 mg/mL; about 35 mg/mL; about 40 mg/mL; about 45 mg/mL; about 50 mg/mL; about 55 mg/mL; about 60 mg/mL; about 65 mg/mL; about 70 mg/mL; about 75 mg/mL; about 80 mg/mL; about 85 mg/mL; about 86 mg/mL; about 87 mg/mL; about 88 mg/mL; about 89 mg/mL; about 90 mg/mL; about 95 mg/mL; about 100 mg/mL; about 105 mg/mL; about 110 mg/mL; about 115 mg/mL; about 120 mg/mL; about 125 mg/mL; about 130 mg/mL; about 131 mg/mL; about 132 mg/mL; about 133 mg/mL; about 134 mg/mL; about 135 mg/mL; about 140 mg/mL; about 145 mg/mL; about 150 mg/mL; about 155 mg/mL; about 160 mg/mL; about 165 mg/mL; about 170 mg/mL; about 175 mg/mL; about 180 mg/mL; about 185 mg/mL; about 190 mg/mL; about 195 mg/mL; about 200 mg/mL; about 205 mg/mL; about 210 mg/mL; about 215 mg/mL; about 220 mg/mL; about 225 mg/mL; about 230 mg/mL; about 235 mg/mL; about 240 mg/mL; about 245 mg/mL; about 250 mg/mL; about 255 mg/mL; about 260 mg/mL; about 265 mg/mL; about 270 mg/mL; about 275 mg/mL; about 280 mg/mL; about 285 mg/mL; about 200 mg/mL; about 200 mg/mL; or about 300 mg/mL.

As described above, the therapeutic protein may be an antigen-binding protein, such as an antibody, an antibody fragment, a trap molecule or other receptor Fc-fusion protein, soluble receptors, and the like. In one particular embodiment, the therapeutic protein is aflibercept, a VEGF antagonist trap molecule. Feedstock solutions for the formation of aflibercept-containing microparticles may contain from about 1 mg/mL to about 100 mg/mL aflibercept, about 2 mg/mL, about 3 mg/mL, about 4 mg/mL, about 5 mg/mL, about 6 mg/mL, about 7 mg/mL, about 8 mg/mL, about 9 mg/mL, about 10 mg/mL, about 15 mg/mL, about 20 mg/mL, about 25 mg/mL, about 30 mg/mL, about 35 mg/mL, about 40 mg/mL, about 45 mg/mL, about 50 mg/mL, about 55 mg/mL, about 60 mg/mL, about 65 mg/mL, about 70 mg/mL, about 75 mg/mL, about 80 mg/mL, about 85 mg/mL, about 90 mg/mL, about 95 mg/mL, or about 100 mg/mL VEGF Trap protein. Solutions may contain one or more buffers of from about 5 mM to about 50 mM. In one embodiment, the buffer is about 10 mM phosphate at a pH of about 6 ± 0.5.

In some embodiments, the protein microparticles of the subject formulated pharmaceutical powder are coated with a polymer. The term "polymer" includes macromolecules comprising repeating monomers connected by covalent chemical bonds. Useful polymers for therapeutic microparticles are biocompatible and biodegradable. A biocompatible or biodegradable polymer can be natural or synthetic. Natural polymers include polynucleotides, polypeptides, such as naturally occurring proteins, recombinant proteins, gelatin, collagens, fibrins, fibroin, polyaspartates, polyglutamates, polyleucine, leucine-glutamate co-polymers; and polysaccharides, such as cellulose alginates, dextran and dextran hydrogel polymers, amylose, inulin, pectin and guar gum, chitosan, chitin, heparin, and hyaluronic acid. Synthetic biocompatible or biodegradable polymers include polylactic acid (PLA), polyglycolic acid (PGA), polylactic-polyglycolic copolymer (PLGA), poly-D,L-lactide-co-glycolide (PLGA), PLGA-ethylene oxide fumarate, PLGA-alpha-tocopheryl succinate esterified to polyethylene glycol 1000 (PLGA-TGPS), polyanhydride poly[1,6-bis(p-carboxyphenoxy)hexane] (pCPH), poly(hydroxbutyric acid-cohydroxyvaleric acid) (PHB-PVA), polyethylene glycol-poly (lactic acid) copolymer (PEG-PLA), poly-ε-caprolactone (PCL), poly-alkyl-cyano-acrylate (PAC), poly(ethyl)cyanoacrylate (PEC), polyisobutyl cyanoacrylate, poly-N-(2-hydroxypropyl)methacrylamide (poly(HPMA)), poly-β-R-hydroxy butyrate (PHB), poly-□-R-hydroxy alkanoate (PHA), poly-β-R-malic acid, phospholipid-cholesterol polymers, 2-dioleoyl-sn-glycero-3-phosphatidylcholine/ polyethyleneglycol-distearoylphosphatidylehtanolamine (DOPC/PEG-DSPE)/Cholesterol, ethyl cellulose, cyclodextrin (CD)-based polyrotaxanes and polypseudorotaxanes, polybutylene succinate (PBS), polyorthoesters, polyorthoester-polyamidine copolymers, polyorthoester-diamine copolymers, polyorthoesters incorporating latent acids tom control rates of degradation, and inter alia poly(ethylene glycol)/poly(butylene terephthalate) copolymers.

Ethyl cellulose (EC) is a well-known and readily available biomaterial used in the pharmaceutical and food sciences. It is a cellulose derivative in which some of the glucose hydroxyl groups are replaced with ethyl ether. Martinac et al., 22(5) J. Microencapsulation 549-561 (2005) is mentioned herein for using ethyl cellulose as a biocompatible polymer in the manufacture of microspheres. US 4,210,529 is mentioned herein for ethyl cellulose and derivatives of ethyl cellulose.

Poly-D,L-lactide-co-glycolide (PLGA) is also a well-known Food and Drug Administration (FDA) approved biocompatible and biodegradable polymer used in tissue engineering and pharmaceutical delivery systems. PLGA is a polyester comprising glycolic acid and lactic acid monomers. Astete and Sabliov, 17(3) Biomater. Sci. Polym. Ed. 247-89 (2006) is mentioned herein for the synthesis of PLGA and the manufacture of PLGA nanoparticles.

Poly-ε-caprolactone (PCL) is another biocompatible and biodegradable polymer approved by the FDA for use in humans as a drug delivery device. PCL is a polyester of ε-caprolactone, which hydrolyses rapidly in the body to form a non-toxic or low toxicity hydroxycarboxylic acid. Labet and Thielemans, 38 Chemical Society Reviews 3484-3504 (2009) is mentioned herein for the manufacture of PCL. Sinha et al., 278(1) Int. J. Pharm. 1-23 (2004)) is mentioned herein for the manufacture of PCL-based microspheres and nanospheres.

Polyorthoester (POE) is a bioerodible polymer designed for drug delivery. It is generally a polymer of a ketene acetal, preferably a cyclic diketene acetal, such as e.g., 3,9-dimethylene-2,4,8,10-tetraoxa spiro[5.5]-undecane, which is polymerized via glycol condensation to form the orthoester linkages. Polyorthoesters can be modified to control their drug release profile and degradation rates by swapping in or out various hydrophobic diols and polyols, such as e.g., replacing a hexanetriol with a decanetriol, as well as adding latent acids, such as *e.g.,* octanedioic acid or the like, to the backbone to increase pH sensitivity. Other modifications to the polyorthoester include the integration of an amine to increase functionality. US 5,968,543; US 4,764,364; US 4,304,767; Heller and Barr, 5(5) Biomacromolecules 1625-32 (2004); and Heller, 57 Adv. Drug. Deliv. Rev. 2053-62 (2005) are mentioned herein for polyorthoesters.

Polyethylene glycol (PEG) can be crosslinked to form a gel, into which the protein microparticles are incorporated. Gibas and Janik, "Review: synthetic polymer hydrogels for biomedical applications," 4(4) Chemistry & Chemical Technology 297-304 (2010) is mentioned herein for crosslinked PEG gels.

Polymers that are preferred for applications that apply heat when spray drying have high glass transition temperatures. In some embodiments, a high glass transition temperature is ≥ 10°C, ≥ 15°C, ≥ 20°C, ≥ 25°C, ≥ 30°C, ≥ 35°C, ≥ 45°C or ≥ 30°C.s

In one aspect, the invention provides a method of manufacturing the subject formulated pharmaceutical powder by "spray drying" the precursor aqueous solution containing the subject protein and any additional excipients (a.k.a. "feedstock" as herein described). The term "spray-dry" means a method of producing a powder comprising micron-sized particles from a solution, slurry or suspension by using a spray-dryer. Spray dryers employ an atomizer or spray nozzle to disperse the suspension or slurry into a controlled drop size spray. Drop sizes from 10 to 500 µm can be generated by spray-drying. As the solvent (water or organic solvent) dries, the protein substance dries into a micron-sized particle *(i.e.,* protein microparticle), forming a powder-like substance; or in the case of a protein microparticle-polymer suspension, forming a polymer hardened shell around the protein load. Spray-drying may be performed on equipment such as *e.g.,* a BUCHI Mini Spray Dryer B-290 (Büchi Labortechnik AG, Flawil, CH). Elversson & Millqvist-Fureby, "Aqueous two-phase systems as a formulation concept for spray-dried protein," 294(1,2) International Journal of Pharmaceutics 73-87 (2005) is mentioned herein by reference to spray-drying.

In one particular embodiment, the feedstock is pumped into the spray dryer at a rate of about 2 mL/min to about 15 mL/min, or about 7 mL/min. In one embodiment, the nozzle pressure is about 35 psi to about 100 psi. In one embodiment, the nozzle pressure is about 75 psi. The inlet temperature of the spray dryer is set at a temperature at or above the boiling point of water, such as e.g., at about 100°C to about 130°C. The outlet temperature at a temperature below the boiling point of water and above ambient temperature, such as e.g., about 55°C. In one specific embodiment, a protein solution (e.g., VEGF Trap solution or IgG solution) is pumped into a BÜCHI Mini Spray Dryer B-290 at about 7 mL/min, with an inlet temperature of about 100°C, 110°C, 120°C, or 130°C and an outlet temperature of about 55°C, with the aspirator set at 33 m3/h and the spray gas at 530 L/h.

In one embodiment, the formulated pharmaceutical powder is formed by subjecting the subject feedstock to atomization to form a mist of atomized droplets, and then applying heat to the mist of atomized droplets to form the formulated pharmaceutical powder comprising the protein. In some embodiments, the resultant formulated pharmaceutical powder is subjected to additional drying (a.k.a. "secondary drying"). Additional drying includes baking, lyophilizing, and nitrogen air-flow. In other embodiments, the resultant formulated pharmaceutical powder is not subjected to additional drying. In some embodiments, the resultant formulated pharmaceutical powder is not subjected to subsequent baking. In some embodiments, the resultant formulated pharmaceutical powder is not subjected to subsequent lyophilization. In some embodiments, the resultant formulated pharmaceutical powder is not subjected to subsequent drying by nitrogen air-flow.

In one embodiment, the feedstock comprises a thermal stabilizer and a glycoprotein with a mass ratio at or between 1:50-2:5. In one embodiment, for every 5 parts of protein by weight the feedstock contains ≤ 2 parts of thermal stabilizer by weight. For example, if the feedstock contains 5 mg/ml of protein, then the totality of the thermal stabilizer would be included at ≤ 2 mg/ml to maintain the ratio of 5 parts protein to ≤ 2 parts thermal stabilizer in the subject formulated pharmaceutical powder, as described herein above. In one embodiment, for every 5 parts of protein by weight the feedstock contains ≤ 1 part of thermal stabilizer by weight. For example, if the feedstock 5 mg/ml of protein, then the totality of the thermal stabilizer would be included at ≤ 1 mg/ml to maintain the ratio of 5 parts protein by weight to 1 part thermal stabilizer by weight in the subject formulated pharmaceutical powder, as described herein above. In some embodiments, the weight to weight ratio of protein to thermal stabilizer is 5:2 - 100:1, 5:2 - 10:3, 20:7 - 4:1, 10:3 - 5:1, 4:1 - 20:3, 5:1 - 10:1, 20:3 - 20:1, 10:1 - 40:1, 20:1 - 50:1, or 40:1 - 100:1.

In another embodiment, for every mole of protein the feedstock contains < 300 moles of thermal stabilizer. For example, if the feedstock contains 1 mM of protein, then the totality of the thermal stabilizer would be included at < 300 mM to maintain the molar ratio of thermal stabilizer to protein of < 300:1 in the resultant subject formulated pharmaceutical powder, as described herein above. In some embodiments, the molar ratio of thermal stabilizer to protein is 350:1 - 1:1, 350:1 - 300:1, 325:1 - 275:1, 300:1 - 250:1, 275:1 - 225:1, 250:1 - 200:1, 225:1-175:1, 200:1 - 150:1, 175:1 - 125:1, 150:1 - 100:1, 125:1 - 75:1, 100:1 - 50:1, 75:1 - 25:1, or 50:1 - ≤ 1:1.

Disclosed, but not claimed, is that the feedstock contains the subject protein without a thermal stabilizer.

Disclosed, but not claimed, is that the resultant formulated pharmaceutical powder is bone dry, as described herein above.

In another embodiment, the resultant formulated pharmaceutical powder is not bone dry, mas described herein above. In some embodiments, the not bone dry formulated pharmaceutical powder comprises between about 3% and about 10% (w/w) water, 3.5%-10% (w/w) water, 4%-10% (w/w) water, 4.5%-10% (w/w) water, 5%-10% (w/w) water, 5.5%-10% (w/w) water, 6%-10% (w/w) water, 6.5%-10% (w/w) water, 7%-10% (w/w) water, 7.5%-10% (w/w) water, 8%-10% (w/w) water, 8.5%-10% (w/w) water, 9%-10% (w/w) water, 9.5%-10% (w/w) water, >3%-9% (w/w) water, >3%-9% (w/w) water, >3%-8.5% (w/w) water, >3%-8% (w/w) water, >3%-7.5% (w/w) water, >3%-7% (w/w) water, >3%-6.5% (w/w) water, >3%-6% (w/w) water, >3%-5.5% (w/w) water, >3%-5% (w/w) water, >3%-4.5% (w/w) water, >3%-4% (w/w) water, >3%-3.5% (w/w) water, about 3.1% (w/w) water, about 3.5% (w/w) water, about 4% (w/w) water, about 4.5% (w/w) water, about 5% (w/w) water, about 5.5% (w/w) water, about 6% (w/w) water, about 6.5% (w/w) water, about 7% (w/w) water, about 7.5% (w/w) water, about 8% (w/w) water, about 8.5% (w/w) water, about 9% (w/w) water, about 9.5% (w/w) water or about 10% (w/w) water.

In some embodiments, the thermal stabilizer of the subject feedstock comprises sucrose or trehalose, or a combination of trehalose and sucrose.

In other embodiments, the thermal stabilizer of the subject feedstock does not comprise a molecule with a molecular mass greater than 200 g/mol. In some embodiments, the thermal stabilizer of the subject feedstock is selected from the group consisting of mannitol, isoleucine, proline, and combinations thereof.

In other embodiments, the thermal stabilizer of the subject feedstock comprises trehalose or sucrose combined with mannitol, isoleucine or proline.

In one embodiment, the subject feedstock also contains a buffer. Preferred buffers include phosphate, acetate and histidine. In one embodiment, the feedstock comprises 0.5 mM - 10 mM of a buffer.

In one embodiment the subject feedstock does not contain a buffer. Here, the feedstock is buffered by the subject protein itself and the water.

In one embodiment, the subject feedstock also contains a non-ionic detergent. Non-ionic detergents include polysorbates such as polysorbate 20 and polysorbate 80. In one embodiment, the nonionic surfactant is present in the feedstock at a concentration of 0.01 - 0.2% (w/v).

In some embodiments, the subject feedstock contains the subject protein, one or more of (i) buffer(s), (ii) thermal stabilizer(s), (iii) and/or surfactant(s). In one embodiment, the aqueous solution contains 2 - 200 mg/ml of the protein, 0.5 - 2% (w/v) of thermal stabilizer(s), 1-10 mM buffer(s), and 0.005 - 0.3% (w/v) of surfactant(s). In a specific embodiment, the aqueous solution comprises 50 mg/ml of the protein, 2% w/v of one or more thermal stabilizers, 10 mM buffer, and 0.015% to 0.1% w/v of one or more nonionic surfactants. In another specific embodiment, the aqueous solution comprises 5 mg/ml of the protein, 0.2% w/v of one or more thermal stabilizers, 1 mM buffer, and 0.015% to 0.1% w/v of one or more nonionic surfactants.

In another embodiment, the powder-constituent protein microparticles formed from the subject feedstock are subsequently coated with a biodegradable polymer. In some embodiments, the subject formulated pharmaceutical powder is suspended in a polymer solution. In one embodiment, the polymer solution is formed by dissolving the polymer in an organic polymer such as methylene chloride, tetrahydrofuran, ethyl acetate, dichloromethane, ethanol, or some other useful solvent. Ethyl acetate is widely known as a safe solvent and is often used in the preparation of drugs, implants and foodstuffs.

In some embodiments, the polymer can be ethyl cellulose ("EC"), poly(lactic acid) ("PLA"), polyorthoester ("POE"), poly-D,L-lactide-co-glycolide ("PLGA"), or poly-ε-caprolactone ("PCL"). In some embodiments, the polymer is polylactic acid (PLA), polyglycolic acid (PGA), crosslinked polyethylene glycol (PEG), poly-D,L-lactide-co-glycolide (PLGA), PLGA-ethylene oxide fumarate, PLGA-alpha-tocopheryl succinate esterified to polyethylene glycol 1000 (PLGA-TGPS), polyanhydride poly[1,6-bis(p-carboxyphenoxy)hexane] (pCPH), poly(hydroxbutyric acid-cohydroxyvaleric acid) (PHB-PVA), polyethylene glycol-poly (lactic acid) copolymer (PEG-PLA), poly-ε-caprolactone (PCL), poly-alkyl-cyano-acrylate (PAC), poly(ethyl)cyanoacrylate (PEC), polyisobutyl cyanoacrylate, poly-N-(2-hydroxypropyl)methacrylamide (poly(HPMA)), poly-β-R-hydroxy butyrate (PHB), poly-β-R-hydroxy alkanoate (PHA), poly-β-R-malic acid, phospholipid-cholesterol polymers, 2-dioleoyl-sn-glycero-3-phosphatidylcholine/ polyethyleneglycol-distearoylphosphatidylehtanolamine (DOPC/PEG-DSPE)/Cholesterol, polysaccharides, cellulose, ethyl cellulose, methyl cellulose, alginates, dextran and dextran hydrogel polymers, amylose, inulin, pectin and guar gum, chitosan, chitin, heparin, hyaluronic acid, cyclodextrin (CD)-based polyrotaxanes and polypseudorotaxanes, polyaspartates, polyglutamates, polylucine, leucine-glutamate co-polymers, polybutylene succinate (PBS), gelatin, collagens, fibrins, fibroin, polyorthoesters, polyorthoester-polyamidine copolymer, polyorthoester-diamine copolymers, polyorthoesters incorporating latent acids, polyethylene glycol)/poly(butylene terephthalate) copolymer, and combinations and copolymers thereof.

The polymer can be dissolved in the solvent (e.g., ethyl acetate) at a concentration of from about 10 mg/mL to about 300 mg/mL (i.e., 1% - 30% [w/v]), from about 15 mg/mL to about 295 mg/mL, from about 20 mg/mL to about 290 mg/mL, from about 25 mg/mL to about 280 mg/mL, from about 30 mg/mL to about 270 mg/mL, from about 35 mg/mL to about 265 mg/mL, from about 40 mg/mL to about 260 mg/mL, from about 45 mg/mL to about 260 mg/mL, from about 50 mg/mL to about 255 mg/mL, from about 55 mg/mL to about 250 mg/mL, about 20 mg/mL, about 25 mg/mL, about 30 mg/mL, about 35 mg/mL, about 40 mg/mL, about 45 mg/mL, about 50 mg/mL, about 75 mg/mL, about 100 mg/mL, about 125 mg/mL, about 150 mg/mL, about 175 mg/mL, about 200 mg/mL, about 225 mg/mL, or about 250 mg/mL. In one embodiment, the polymer concentration in the inner feed of the spray dryer is ≤ 10% (w/v) and the polymer concentration of the outer feed is ≤ 25% (w/v).

The subject formulated protein powder is then added to the polymer solution at about 10 mg/mL to about 100 mg/mL, about 15 mg/mL to about 95 mg/mL, about 20 mg/mL to about 90 mg/mL, about 25 mg/mL to about 85 mg/mL, about 30 mg/mL to about 80 mg/mL, about 35 mg/mL to about 75 mg/mL, about 40 mg/mL to about 70 mg/mL, about 45 mg/mL to about 65 mg/mL, about 50 mg/mL to about 60 mg/mL, at about 25 mg/mL, at about 30 mg/mL, at about 35 mg/mL, at about 40 mg/mL, at about 45 mg/mL, or at about 50 mg/mL. The powder and polymer solution are mixed to form a slurry or suspension, which is then subjected to dispersion and drying to form the polymer coated formulated pharmaceutical powder. The preferred polymer solution contains the polymer dissolved in a solvent that does not dissolve the suspended protein particles or its excipients. For example, in those embodiments in which the powder contains sucrose as an excipient, ethanol is not preferred because sucrose can dissolve into ethanol and leave behind the protein in the particle which his insoluble.

In one embodiment, the powder and polymer slurry or suspension is subjected to spray drying, which is performed in a manner similar to the method for manufacturing the subject formulated pharmaceutical powder, but with a reduced intake temperature (Tin) to protect against igniting the organic solvent or polymer. For example, when the organic solvent is dichloromethane, the Tᵢₙ may be 40°C; when the solvent is ethyl acetate, the Tᵢₙ may be 77°C; and when the solvent is ethanol, the Tᵢₙ may be 78°C. Briefly, the powder and polymer slurry or suspension is pumped into the spray dryer at a rate of about 0.5 mL/min to about 20 mL/min, about 1.2 mL/min, about 2.6 mL/min, or about 12.5 mL/min. In one embodiment, a dual-feed nozzle spray dryer is used to atomize the slurry. In one embodiment, the viscosity of the polymer solution is < 20 cPoise at 20°C. In one embodiment, the polymer concentration of the inner feed is ≤ 10% (w/v) and of the polymer concentration of the outer feed is ≤ 25%. In one embodiment, the microparticles are suspended in the polymer solution at < 10% (w/v). In one embodiment, the Tᵢₙ is dependent upon the solvent, *e.g.,* 40°C for dichloromethane, 77°C for ethyl acetate, and 78°C for ethanol. In one embodiment, the Tₒᵤₜ is less than the glass transition temperature of the subject formulated pharmaceutical powder and is a function of Tᵢₙ, flow rate, solvent, and the aspirator. In one embodiment, the Tₘₐₓ of the nozzle is < 150°C. In one embodiment, the maximum flow rate of the outer channel is about 1.2 mL/min and the maximum flow rate of the inner channel is about 2.6 mL/min. In one embodiment, the power to the sonicator of the spray drier is about 0.5 - 2 W and the aspirator is set to about 80%.

The resulting polymer coated formulated pharmaceutical powder contains constituent protein microparticles surrounded with a polymer cortex. Such polymer-coated protein microparticles may have any of a variety of architectures. In one embodiment, some polymer-coated protein microparticles contain between 1 and 50, between 1 and 40, between 1 and 30, between 1 and 20, between 1 and 10 or between 1 and 5 protein microparticles embedded in a single polymer-coated protein microparticle. In some embodiments, an individual polymer-coated protein microparticle contains 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44,45,46,47,48,49 or 50 protein microparticles. In some embodiments, the formulated pharmaceutical powder contains a mode polymer-coated protein microparticle with 1-2, 2-3 or 3-4 protein microparticles.

In some embodiments, the polymer-coated protein microparticles have a range of diameters of from about 2 µm to about 70 µm, about 5 µm to about 65 µm, about 10 µm to about 60 µm, about 15 µm to about 55 µm, about 20 µm to about 50 µm, about 15 µm, about 20 µm, about 25 µm, or about 30 µm. The size variation in large part reflects the thickness of the polymer cortex, although the diameter of the protein core could contribute to size variation to some extent. Manipulating the starting concentration of the polymer solution or the polymer itself can be used to control the diameter of the final micron-sized particle.

Disclosed, but not claimed, is that the resultant polymer-coated formulated pharmaceutical powder is subjected to additional drying (a.k.a. "secondary drying"). Additional drying includes baking, lyophilizing, and nitrogen air-flow. In other embodiments, the resultant polymer-coated formulated pharmaceutical powder is not subjected to additional drying. In some embodiments, the resultant polymer-coated formulated pharmaceutical powder is not subjected to subsequent baking. In some embodiments, the resultant polymer-coated formulated pharmaceutical powder is not subjected to subsequent lyophilization. In some embodiments, the resultant polymer-coated formulated pharmaceutical powder is not subjected to subsequent drying by nitrogen air-flow.

The polymer coated microparticles of the instant invention are useful in the time-release or extended release of protein therapeutics. For example, it is envisioned that the aflibercept microparticles are useful in the extended release of aflibercept in the vitreous for the treatment of vascular eye disorders, or subcutaneous implantation for the extended release of VEGF Trap to treat cancer or other disorders.

U.S. Published Patent Application No. US 2013/0129830 A1, which was published on May 23, 2013, and U.S. Provisional Application No. 62/405,610, which was filed on October 7, 2016 are generally mentioned here.

### EXAMPLES

The following examples are put forth so as to provide those of ordinary skill in the art with a description of how to make and use the methods and compositions of the invention, and are not intended to limit the scope of what the inventors regard as their invention.

### Example 1: Spray Dry Parameters

Disclosed is a method of producing a formulated pharmaceutical powder containing a protein via employing a spray drying step to an aqueous feedstock solution containing the protein. A feedstock solution containing 50 mg/mL aflibercept, 10 mM phosphate (pH 6.2), and 2% w/v sucrose was subjected to spray drying using a BUCHI B-290 mini spray dryer (Flawil, CH). Here, inlet temperature was varied from 100°C to 130°C in ten degree increments and the ECD of the resultant particles were determined by MFI. As shown in Figure 1, inlet temperature of the drying gas did not affect the size distribution or morphology of the protein microparticles.

At each inlet temperature, less than one percent of the resultant particles were greater than 10 microns in size. Transient exposure to high temperatures and evaporative cooling prevented the aflibercept protein from degrading while spray drying.

### Example 2: Protein Integrity and Stability

The molecular integrity of aflibercept in the post-spray dry formulated pharmaceutical powder was assessed. Reconstituted aflibercept powder was compared to a liquid aflibercept formulation for ophthalmic injection, and to the precursor feedstock formulation containing aflibercept. The ophthalmic formulation comprised 40 mg/mL aflibercept, 5% (w/v) sucrose, 0.03% (w/v) polysorbate 20, 40 mM NaCl, and 10 mM phosphate, pH 6.2. The pre-spray dried feedstock and reconstituted spray dried formulations comprised 50 mg/mL aflibercept, 2% (w/v) sucrose, and 10 mM phosphate, pH 6.2. The aflibercept in the post-spray dry formulated pharmaceutical powder maintained its nativity and biological potency (see Table 5).

Stability of the aflibercept in the spray dried powder was assessed by measuring the formation of high molecular weight species. The formation of high molecular weight (HMW) species at 37°C at 3 months, 6 months, and 12 months was determined by SE-UPLC of reconstituted protein. Spray dried aflibercept powder was incubated at 37°C under dry conditions and then reconstituted to 50 mg/mL aflibercept for analysis. The rate of formation of HMW species was determined by linear regression of the square root time for time points collected up to 3 months, 6 months, or 12 months at 37°C. The results, which are depicted in Table 6, indicate that the rate of aggregation of spray dried aflibercept is within pharmaceutically acceptable parameters.

**TABLE 5: Integrity of Spray Dried Aflibercept**

| | | Ophthalmic Formulation Aflibercept | Pre-Spray Dried Aflibercept | Reconstituted Spray-Dried Aflibercept |
|---|---|---|---|---|
| % Recovery (RP-HPLC) | | 99 | 100 | 96 |
| Purity (SE-UPLC) | % Native | 99.3 | 99.1 | 99.0 |
| Charge Variant Analysis (iCIEF) | % Main | 79.5 | 79.9 | 79.8 |
| Purity by SDS-PAGE (Non-Reduced) | % Main | 97.0 | 97.5 | 96.9 |
| Purity by SDS-PAGE (Reduced) | % Main | 99.0 | 99.0 | 99.0 |
| mDSC | Tₘ1 (°C) | 67.6 | - | 67.3 |
| | Tₘ2 (°C) | 85.3 | - | 85.6 |
| FTIR | % α-Helix | 8.3 | - | 8.3 |
| | % β-Sheet | 39.2 | - | 39.5 |
| % Relative Potency (Bioassay) | | 84 | 109 | 103 |

**TABLE 6: Stability of Aflibercept Spray Dried Formulated Powder**

| Particle Size | Thermal Stabilizers + Base Formulation (50 mg/mL aflibercept, 10mM phosphate, pH 6.2) | Rate of Aflibercept HMW Species Formation at 37°C (% HMW/√mo by SE-UPLC) |
|---|---|---|
| 5 µm | 1% w/v sucrose | 5.7% |
| 5 µm | 2% w/v sucrose | 3.7% |

### Example 3: Thermal Stabilizers

Adding excipients to the feedstock was shown to improve the thermal stability of microparticulate protein. Lyophilized and spray dried aflibercept *(i.e.,* 2.5 micron particles made from 5 mg/mL aflibercept feedstock) were incubated at 50°C under dry conditions and reconstituted to 5 mg/mL aflibercept for analysis. The results are depicted in Table 7. The powder flowability of formulations containing mannitol and isoleucine appeared similar to the flowability of those formulations containing only sucrose or trehalose. The inclusion of mannitol and isoleucine improved the thermal stability of the spray dried formulations comprising the smaller, *i.e*., 2.5µm particles.

**Table 7: Thermal Stability of 2.5 micron Protein Microparticles**

| Format | Particle Size | Thermal Stabilizers + Base Formulation (5 mg/mL aflibercept, 1 mM phosphate, pH 6.2) | Rate of HMW Aflibercept Species Formation at 50°C (% HMW/√mo by SE-UPLC) |
|---|---|---|---|
| Spray Dried | 2.5 µm | 0.2% w/v sucrose | 8.5% |
| Spray Dried | 2.5 µm | 0.2% w/v trehalose | 8.8% |
| Spray Dried | 2.5 µm | 0.1% w/v sucrose | 5.9% |
| | | 0.05% w/v mannitol | |
| | | 0.05% w/v Ile | |
| Spray Dried | 2.5 µm | 0.1% w/v trehalose | 5.7% |
| | | 0.05% w/v mannitol | |
| | | 0.05% w/v Ile | |
| Spray Dried | 2.5 µm | 0.1% w/v mannitol | 7.0% |
| | | 0.1% w/v Ile | |

### Example 4: Particle Size and Shape

Nozzle inlet temperature, inclusion of surfactant, concentration of aflibercept solute, and inclusion of thermal stabilizers were assessed for their effect on particle size or shape. Protein concentration and the inclusion of surfactant each resulted in smaller - and in the case of surfactant, rounder - microparticles. These results are summarized in Table 8.

**Table 8: Particle Size and Shape**

| Spray Drying Parameter | Effect on Particle Size | Effect on Particle Morphology | Effect on Protein Stability |
|---|---|---|---|
| Inlet Temperature (110-130°C) | None | None | None |
| + Polysorbate 20 (<0.1% w/v) | Decreased | More spherical | None |
| ↓ Solute Concentration | Decreased | None | Increased HMW species (∼0*.*3%)*post* spray dry |
| + Thermal Stabilizer | None | None | Decreased rate of HMW formation under dry thermal stress conditions. Magnitude is dependent on amount and type of thermal stabilizer. |

As described in Example 1, inlet temperature was varied from 100°C to 130°C in ten degree increments and the ECD of the resultant particles were determined by MFI. As shown in Figure 1, inlet temperature of the drying gas did not affect particle size distribution or morphology. At each inlet temperature, less than one percent of the resultant particles were greater than 10 microns in size.

The addition of 0.03%-0.1% w/v polysorbate 20 to the feedstock produced more small particles when spray dried compared to feedstock solutions that did not contain polysorbate 20 (Figure 2). Powder from formulations containing more than 0.1% w/v polysorbate 20 exhibited more agglomerates and was less flowable *(i.e.,* stickier). Low flow powders are difficult to handle in downstream processes. Therefore, formulations containing less than 0.1% w/v polysorbate 20 are preferred. The addition of 0.03% w/v polysorbate 20 to the feedstock solution was also found to produce more spherical particles *(i.e.,* higher aspect ratio [minor axis/major axis]) when spray dried (see Figure 3).

Lower solute concentration in the feedstock solution reduced the spray dried aflibercept particle size to about 2.5µm (compared to 5 µm). The spray drying method using 50 mg/mL aflibercept, 10 mM phosphate (pH 6.2), 2% sucrose (w/v) as the feedstock solution produced particles of ~5 µm diameter. Lowering the solute concentration by 10x in the feedstock solution *(i.e.,* 5 mg/mL aflibercept, 1 mM phosphate [pH 6.2], 0.2% sucrose [w/v]) reduced protein microparticle size by half to -2.5 µm (see Figure 4). This represents an 8-fold decrease in volume of the particle (2³). Spray drying the more dilute feedstock required 10-fold more time (~1 hr/g) to produce the same amount of powder as the higher concentration formulations. The larger surface to volume ratio of the smaller protein microparticles coupled with longer exposure to the outlet temperature during spray drying may stress the protein and may compromise stability to some extent.

### Example 5: Polymer Coating

The protein microparticles of the present invention may be coated with a polymer. This process can be referred to as "spray coating". Briefly, a polyorthoester was dissolved in dichloromethane, ethyl acetate, or ethanol to a viscosity of about < 20 cP at 20°C. As a dual feed nozzle was used (BUCHI B-290 mini spray dryer (Flawil, CH)), the polymer concentration at the inner feed was ≤ 10% (w/v) and at the outer feed ≤ 25% (w/v). The microparticles were suspended in the polymer solution at less than 10% w/v. The Tᵢₙ was 40°C when the solvent was dichloromethane, 77°C when the solvent was ethyl acetate, and 78°C when the solvent was ethanol. The Tₒᵤₜ was kept below the glass transition temperature (T_{g}) by using a thermal jacket filled with circulating ice water to cool down the cyclone. The Tₒᵤₜ was a function of Tᵢₙ, flow rate, the nature of the solvent, and the aspirator. The Tₘₐₓ of the nozzle was kept below 150°C. The maximum flow rates of the outer channel and inner channel of the dual-feed nozzle were 1.2 mL/min and 2.6 mL/min, respectively. The power to the sonicator was from 0.5 W to 2 W; and the aspirator was set to 80%.

A significant shift in the size distribution towards larger particles after spray coating the protein microparticles with polyorthoester (POE) was observed by MFI (see Figure 5).

### Example 6: Protein Specification Comparison

Protein (aflibercept) from reconstituted protein microparticles (Recon DP) was compared to pre-spray dried protein (Pre-SD FDS) and aflibercept prepared as a liquid formulation (EYLEA^{®} DP) to determine any detrimental effects from spray drying. In one experiment, each of these three formulations was subjected to sedimentation velocity - analytical ultracentrifugation (SV-AUC). This method is used to detect and quantify protein aggregates (see Arthus et al., "Detection of protein aggregates by sedimentation velocity analytical ultracentrifugation (SV-AUC): sources of variability and their relative importance," 98(10) J. Pharm. Sci. 3522-39, 2009). SV-AUC showed all lots are comparable in terms of HMW species (Table 9). EYLEA^{®} DP may have a slightly lower aggregate content, whereas reconstituted spray dried aflibercept may have aggregates shifted to larger species. But given the variability among replicate samples, neither of those trends is clearly significant, and overall these results provide no clear or compelling evidence that the three samples are not comparable.

**Table 9: Protein Aggregation Determined by SV-AUC**

| | Monomer sedimentation coefficient, S (Mean ± SD) | % Putative Dimer (7.5-8.0 S) (Mean ± SD) | % Total Aggregates (Mean ± SD) |
|---|---|---|---|
| EYLEA^{®} DP | 5.161 ± 0.004 | 1.3 ± 0.7 | 1.7 ± 1.2 |
| Pre-SD FDS | 5.181 ± 0.014 | 1.5 ± 0.5 | 2.2 ± 1.2 |
| Recon DP | 5.174 ± 0.002 | 1.5 ± 0.5 | 2.3 ± 1.2 |

In another experiment, each of these three formulations was subjected to size exclusion chromatography - multiangle laser light scattering (SEC-MALLS). This method is also used to detect and quantify protein aggregates as well as misfolded proteins (see P. J. Wyatt, "Light scattering and the absolute characterization of macromolecules," 272 Anal. Chim. Acta 1-40, 1993; Odaka et al., "Ligand-Binding Enhances the Affinity of Dimerization of the Extracellular Domain of the Epidermal Growth Factor Receptor1," 122 J. Biochem. 116-121, 1997; J. S. Philo, "Is any measurement method optimal for all aggregate sizes and types?" 8(3) AAPS J. E564-71, 2006). SEC-MALLS showed all lots were comparable with respect to peak area percentages and calculated mass for each species (see Table 10).

**Table 10: Protein Aggregation Determined by SEC-MALLS**

| Sample | Peak 1 (HMW) | | Peak 2 (Main - Native) | |
|---|---|---|---|---|
| | MW (kDa) | Peak Area % | MW (kDa) | Peak Area % |
| EYLEA^{®} DP | 264.2 (0.0) | 1.0 (0.4) | 119.6 (1.0) | 99.0 (0.0) |
| Pre-SD FDS | 264.9 (3.0) | 0.5 (0.0) | 119.9 (0.0) | 99.5 (0.0) |
| Recon DP | 268.1 (6.0) | 1.2 (0.0) | 120.4 (1.0) | 98.8 (0.0) |

The three formulations of aflibercept were also subjected to capillary electrophoresis (CE)-oligosaccharide fingerprinting (see Chen et al., "Profiling glycoprotein n-linked oligosaccharide by capillary electrophoresis," 19(15) Electrophoresis 2639-44, 1998) and tryptic fragment fingerprinting by ultraperformance liquid chromatography - mass spectroscopy (UPLC-MS) (see Sinha et al., "Comparison of LC and LC/MS methods for quantifying N-glycosylation in recombinant IgGs," 19(11) J. Am. Soc. Mass. Spectrom. 1643-54, 2008) to assess glycosylation and other posttranslational modifications, as well as primary sequence. CE-oligosaccharide fingerprinting showed all lots are comparable (Table 11).

**Table 11: Post-Translational Modifications**

| Post-translational Modification | | EYLEA^{®} DP | Pre-SD FDS | Recon DP |
|---|---|---|---|---|
| C-Terminal Peptides w/ and w/o Lys⁴³² | Without Lys ⁴³² | 96.2% | 96.4% | 96.5% |
| | With Lys⁴³² | 3.8% | 3.6% | 3.5% |
| Deamidation (isoAsp) Level at Asn⁸⁴-Gly Motif | Asn⁸⁴ | 78.9% | 79.5% | 79.3% |
| | isoAsp⁸⁴ | 21.1% | 20.5% | 20.7% |
| Oxidation Level at Met¹⁰ | Met¹⁰ | 97.6% | 97.5% | 97.0% |
| | Met(O)¹⁰ | 2.4% | 2.5% | 3.0% |
| Oxidation Level at Met¹⁹² | Met¹⁹² | 96.6% | 96.6% | 96.4% |
| | Met(O)¹⁹² | 3.4% | 3.4% | 3.6% |
| Oxidation Level at Met²³⁷ | Met²³⁷ | 97.2% | 97.2% | 97.2% |
| | Met(O)²³⁷ | 2.8% | 2.8% | 2.8% |
| DYLTHR Peptide Level (related to NR1 band) | ⁹¹DYLTHR⁹⁶ | 0.29% | 0.34% | 0.33% |
| N-Terminal Peptide Glycation at Arg⁵ | Modified N-term | 9.2% | 9.0% | 9.0% |
| Asn⁶⁸ Non-glycosylation Level | Glycosylated | 64.3% | 63.8% | 64.6% |
| | Non-glycosylated | 35.7% | 36.2% | 35.4% |

The peptide map chromatograms under reducing and non-reducing conditions were visually comparable among the three aflibercept samples. No unique or significant peaks corresponding to mutated sequences were observed in the spray dried sample. All post-translational modifications were present in all the samples: C-terminal lysine removal, asparagine deamidation, methionine oxidation, and asparagine glycation. The site-specific glycosylation profiles at the 5 N-linked glycosylation sites are shown in all three samples. The patterns of the disulfide-bonded peptides in all three samples conform to the disulfide bonding patterns expected for aflibercept. No significant levels of either scrambled or free cysteine-containing peptides were identified in the non-reduced peptide maps in the three samples analyzed. The peptide mapping analyses of the spray-dried aflibercept conformed to the EYLEA^{®} DP reference standard.

### Example 7: Effect of Moisture on Stability of Protein in Formulated Pharmaceutical Powders

Spray dry formulations of three different proteins were made according to the process set forth in Example 1. Four formulated powders for each of the three proteins were made with varying amounts of moisture. The three proteins were an IgG1, an IgG4 and a trap molecule (aflibercept). Each aflibercept powder contained 83.8% (w/w) aflibercept, 2.1% (w/w) phosphate, and 14.1% (w/w) sucrose with amounts of water ranging from < 0.5% to > 6%. Each IgG1 powder contained 80.9% (w/w) IgG1, 1.5% (w/w) histidine, 16.4% (w/w) sucrose and 0.2% polysorbate 80 with amounts of water ranging from < 0.5% to > 6%. Each IgG4 powder contained 44.5% (w/w) IgG1, 0.4% (w/w) acetate, 56.3% (w/w) sucrose and 0.4% polysorbate 20 with amounts of water ranging from < 0.5% to > 6%.

The powders were stored at 50°C for up to 6 weeks. Samples were obtained at three weeks, one month and six weeks. The samples were reconstituted and the proteins assessed for change in high molecular weight species by SE-UPLC. The rate of aggregation was calculated based on the HMW values obtained from the reconstituted samples. The results are presented in Table 12.

**Table 12: Effect of Moisture in Formulated Pharmaceutical Powder on Protein Stability**

| Molecule | Moisture Content (% water w/w) | Rate of Aggregation at 50°C (% HMW/mo^{-½}) |
|---|---|---|
| IgG4 | 0.36 | 1.08 |
| | 0.80 | 1.08 |
| | 3.71 | 1.38 |
| | 12.94 | 11.62 |
| IgG1 | 0.01 | 12.60 |
| | 0.88 | 11.07 |
| | 5.31 | 8.74 |
| | 13.06 | 20.84 |
| aflibercept | 0.01 | 13.29 |
| | 0.92 | 12.47 |
| | 5.45 | 10.99 |
| | 14.12 | 27.20 |

## Claims

1. A method of manufacturing a formulated pharmaceutical powder, the method comprising:
a. atomizing an aqueous solution via a spray dryer, the aqueous solution comprising:
i. 0.1%-2.0% (w/v) of a thermal stabilizer and 50 mg/mL to 180 mg/mL of a protein with a mass ratio at or between 1:5-2:5, wherein the thermal stabilizer is selected from sucrose, trehalose, mannitol, isoleucine, proline, or combinations thereof and the protein is (a) a Fc containing glycoprotein selected from an antibody or a receptor Fc fusion protein, or (b) a soluble receptor, and
ii. 0.01-0.2% (w/v) of a nonionic surfactant selected from the group consisting of alkyl polyethylene oxide), alkyl polyglucosides, fatty alcohols, cocamide MEA, cocamide DEA, cocamide TEA, polyoxyethylene sorbitan esters, poloxamers, poloxamer 188, poloxamer 407, polyethylene-polypropylene glycol, and polyethylene glycol,
wherein an inlet temperature of the spray dryer is set at a temperature of 100°C to 130°C and an outlet temperature of the spray dryer is set at a temperature that is below the boiling point of water and above ambient temperature; and
b. applying heat to the atomized aqueous solution to form the formulated pharmaceutical powder, wherein the formulated pharmaceutical powder is not subjected to secondary drying, wherein the formulated pharmaceutical powder comprises between 3% to 10 (w/w) water.

2. The method of claim 1, wherein the receptor Fc fusion protein is a trap protein.

3. The method of claim 1 or 2, wherein the protein is a soluble receptor.

4. The method of any one of claims 1 to 3, wherein the outlet temperature of the spray dryer is set at 55 °C.

5. The method of claim 1, further comprising:
suspending the formulated pharmaceutical powder in an organic solution comprising a biodegradable polymer; and
spray drying the suspension to form a coated formulated pharmaceutical powder, wherein the formulated pharmaceutical powder is not subjected to secondary drying prior to forming the coated formulated pharmaceutical powder.

6. A method of manufacturing a formulated pharmaceutical powder, the method comprising:
a. atomizing an aqueous solution via a spray dryer, the aqueous solution comprising:
i. 0.1%-2.0% (w/v) of a thermal stabilizer and 50 mg/mL to 180 mg/mL of a protein at a molar ratio of less than 300 moles of the thermal stabilizer per mole of the protein, wherein the thermal stabilizer is selected from sucrose, trehalose, mannitol, isoleucine, proline, or combinations thereof, wherein and protein is (a) a Fc containing glycoprotein selected from an antibody or a receptor Fc fusion protein, or (b) a soluble receptor, and
ii. 0.01-0.2% (w/v) of a nonionic surfactant selected from the group consisting of alkyl polyethylene oxide), alkyl polyglucosides, fatty alcohols, cocamide MEA, cocamide DEA, cocamide TEA, polyoxyethylene sorbitan esters; poloxamers, poloxamer 188, poloxamer 407, polyethylene-polypropylene glycol, and polyethylene glycol,
wherein an inlet temperature of the spray dryer is set at a temperature of 100°C to 130°C and an outlet temperature of the spray dryer is set at a temperature that is below the boiling point of water and above ambient temperature; and
b. applying heat to the atomized aqueous solution to form the formulated pharmaceutical powder, wherein the formulated pharmaceutical powder is not subjected to secondary drying, wherein the formulated pharmaceutical powder comprises between 3% to 10% (w/w) water.

7. The method of claim 6, wherein the receptor Fc fusion protein is a trap protein.

8. The method of claim 6 or 7, wherein the protein is a soluble receptor.

9. The method of any one of claims 6 to 8, wherein the outlet temperature of the spray dryer is set at 55 °C.

10. The method of claim 6, further comprising:
suspending the formulated pharmaceutical powder in an organic solution comprising a biodegradable polymer; and
spray drying the suspension to form a coated formulated pharmaceutical powder, wherein the formulated pharmaceutical powder is not subjected to secondary drying prior to forming the coated formulated pharmaceutical powder.

11. A formulated pharmaceutical powder comprising:
60%-97% (w/w) of a glycoprotein, and 3%-40% (w/w) of a thermal stabilizer, wherein the glycoprotein is an antibody or a receptor Fc fusion protein, and wherein the thermal stabilizer is selected from sucrose, trehalose, mannitol, isoleucine, proline, or combinations thereof;
0.01-0.2% (w/v) of a nonionic surfactant selected from the group consisting of alkyl poly(ethylene oxide), alkyl polyglucosides, fatty alcohols, cocamide MEA, cocamide DEA, cocamide TEA, polyoxyethylene sorbitan esters; poloxamers, poloxamer 188, poloxamer 407, polyethylene-polypropylene glycol, and polyethylene glycol; and
3%-10% (w/w) water, wherein the formulated pharmaceutical powder is not subjected to secondary drying, and wherein the aggregation rate is less than 5% per month Λ ½

12. The formulated pharmaceutical powder of claim 11, wherein the receptor Fc protein is trap protein.

13. The method of any one of claims 1 to 10 or the formulated powder of claim 11 or 12, wherein the alkyl polyglucoside is selected from octyl glucoside and decyl maltoside.

14. The method of any one of claims 1 to 10 or the formulated powder of claim 11 or 12, wherein the fatty alcohols are selected from cetyl alcohol and oleyl alcohol.

15. The method of any one of claims 1 to 10 or the formulated powder of claim 11 or 12, wherein the polyoxyethylene sorbitan ester is selected from polysorbate 20, polysorbate 28, polysorbate 40, polysorbate 60, polysorbate 65, polysorbate 80, polysorbate 81, and polysorbate 85.

## Patentansprüche

1. Verfahren zur Herstellung eines formulierten pharmazeutischen Pulvers, wobei das Verfahren Folgendes umfasst:
a. Zerstäuben einer wässrigen Lösung über einen Sprühtrockner, wobei die wässrige Lösung Folgendes umfasst:
i. 0,1-2,0 % (Gew./Vol.) eines Thermostabilisators und 50 mg/ml bis 180 mg/ml eines Proteins mit einem Massenverhältnis von oder zwischen 1:5-2:5, wobei der Thermostabilisator ausgewählt ist aus Saccharose, Trehalose, Mannit, Isoleucin, Prolin oder Kombinationen davon und das Protein ist (a) ein Fc-haltiges Glykoprotein, das ausgewählt ist aus einem Antikörper oder einem Rezeptor-Fc-Fusionsprotein, oder (b) ein löslicher Rezeptor, und
ii. 0,01-0,2 % (Gew./Vol.) eines nichtionischen Tensids, das ausgewählt ist aus der Gruppe bestehend aus Alkylpoly(ethylenoxid), Alkylpolyglucosiden, Fettalkoholen, Cocamid-MEA, Cocamid-DEA, Cocamid-TEA, Polyoxyethylensorbitanestern, Poloxameren, Poloxamer 188, Poloxamer 407, Polyethylen-Polypropylenglykol und Polyethylenglykol,
wobei eine Einlasstemperatur des Sprühtrockners auf eine Temperatur von 100 °C bis 130 °C und eine Auslasstemperatur des Sprühtrockners auf eine Temperatur eingestellt ist, die unter dem Siedepunkt von Wasser und über Umgebungstemperatur liegt; und
b. Anwenden von Wärme auf die zerstäubte wässrige Lösung, um das formulierte pharmazeutische Pulver zu bilden, wobei das formulierte pharmazeutische Pulver keiner Sekundärtrocknung unterzogen wird, wobei das formulierte pharmazeutische Pulver zwischen 3 % und 10 (Gew./Gew.) Wasser umfasst.

2. Verfahren nach Anspruch 1, wobei das Rezeptor-Fc-Fusionsprotein ein Trap-Protein ist.

3. Verfahren nach Anspruch 1 oder 2, wobei das Protein ein löslicher Rezeptor ist.

4. Verfahren nach einem der Ansprüche 1 bis 3, wobei die Auslasstemperatur des Sprühtrockners auf 55 °C eingestellt ist.

5. Verfahren nach Anspruch 1, ferner umfassend:
Suspendieren des formulierten pharmazeutischen Pulvers in einer organischen Lösung, die ein biologisch abbaubares Polymer umfasst; und
Sprühtrocknen der Suspension, um ein beschichtetes formuliertes pharmazeutisches Pulver zu bilden, wobei das formulierte pharmazeutische Pulver vor der Bildung des beschichteten formulierten pharmazeutischen Pulvers keiner Sekundärtrocknung unterzogen wird.

6. Verfahren zur Herstellung eines formulierten pharmazeutischen Pulvers, wobei das Verfahren Folgendes umfasst:
a. Zerstäuben einer wässrigen Lösung über einen Sprühtrockner, wobei die wässrige Lösung Folgendes umfasst:
i. 0,1-2,0 % (Gew./Vol.) eines Thermostabilisators und 50 mg/ml bis 180 mg/ml eines Proteins in einem Molverhältnis von weniger als 300 Mol des Thermostabilisators pro Mol des Proteins, wobei der Thermostabilisator ausgewählt ist aus Saccharose, Trehalose, Mannit, Isoleucin, Prolin oder Kombinationen davon, wobei das Protein (a) ein Fc-haltiges Glykoprotein ist, das ausgewählt ist aus einem Antikörper oder einem Rezeptor-Fc-Fusionsprotein, oder (b) ein löslicher Rezeptor ist, und
ii. 0,01-0,2 % (Gew./Vol.) eines nichtionischen Tensids, das ausgewählt ist aus der Gruppe bestehend aus Alkylpoly(ethylenoxid), Alkylpolyglucosiden, Fettalkoholen, Cocamid-MEA, Cocamid-DEA, Cocamid-TEA, Polyoxyethylensorbitanestern; Poloxameren, Poloxamer 188, Poloxamer 407, Polyethylen-Polypropylenglykol und Polyethylenglykol,
wobei eine Einlasstemperatur des Sprühtrockners auf eine Temperatur von 100 °C bis 130 °C und eine Auslasstemperatur des Sprühtrockners auf eine Temperatur eingestellt ist, die unter dem Siedepunkt von Wasser und über Umgebungstemperatur liegt; und
b. Anwenden von Wärme auf die zerstäubte wässrige Lösung, um das formulierte pharmazeutische Pulver zu bilden, wobei das formulierte pharmazeutische Pulver keiner Sekundärtrocknung unterzogen wird, wobei das formulierte pharmazeutische Pulver zwischen 3 % und 10 % (Gew./Gew.) Wasser umfasst.

7. Verfahren nach Anspruch 6, wobei das Rezeptor-Fc-Fusionsprotein ein Trap-Protein ist.

8. Verfahren nach Anspruch 6 oder 7, wobei das Protein ein löslicher Rezeptor ist.

9. Verfahren nach einem der Ansprüche 6 bis 8, wobei die Auslasstemperatur des Sprühtrockners auf 55 °C eingestellt ist.

10. Verfahren nach Anspruch 6, ferner umfassend:
Suspendieren des formulierten pharmazeutischen Pulvers in einer organischen Lösung, die ein biologisch abbaubares Polymer umfasst; und
Sprühtrocknen der Suspension, um ein beschichtetes formuliertes pharmazeutisches Pulver zu bilden, wobei das formulierte pharmazeutische Pulver vor der Bildung des beschichteten formulierten pharmazeutischen Pulvers keiner Sekundärtrocknung unterzogen wird.

11. Formuliertes pharmazeutisches Pulver, umfassend:
60-97 % (Gew./Gew.) eines Glykoproteins und 3-40 % (Gew./Gew.) eines Thermostabilisators, wobei das Glykoprotein ein Antikörper oder ein Rezeptor-Fc-Fusionsprotein ist und wobei der Thermostabilisator ausgewählt ist aus Saccharose, Trehalose, Mannit, Isoleucin, Prolin oder Kombinationen davon;
0,01-0,2 % (Gew./Vol.) eines nichtionischen Tensids, das ausgewählt ist aus der Gruppe bestehend aus Alkylpoly(ethylenoxid), Alkylpolyglucosiden, Fettalkoholen, Cocamid-MEA, Cocamid-DEA, Cocamid-TEA, Polyoxyethylensorbitanestern; Poloxameren, Poloxamer 188, Poloxamer 407, Polyethylen-Polypropylenglykol und Polyethylenglykol; und
3-10 % (Gew./Gew.) Wasser, wobei das formulierte pharmazeutische Pulver keiner Sekundärtrocknung unterzogen wird und wobei die Aggregationsrate weniger als 5 % pro Monat Λ ½ beträgt.

12. Formuliertes pharmazeutisches Pulver nach Anspruch 11, wobei das Rezeptor-Fc-Protein ein Trap-Protein ist.

13. Verfahren nach einem der Ansprüche 1 bis 10 oder formuliertes Pulver nach Anspruch 11 oder 12, wobei das Alkylpolyglucosid aus Octylglucosid und Decylmaltosid ausgewählt ist.

14. Verfahren nach einem der Ansprüche 1 bis 10 oder formuliertes Pulver nach Anspruch 11 oder 12, wobei die Fettalkohole aus Cetylalkohol und Oleylalkohol ausgewählt sind.

15. Verfahren nach einem der Ansprüche 1 bis 10 oder formuliertes Pulver nach Anspruch 11 oder 12, wobei der Polyoxyethylensorbitanester ausgewählt ist aus Polysorbat 20, Polysorbat 28, Polysorbat 40, Polysorbat 60, Polysorbat 65, Polysorbat 80, Polysorbat 81 und Polysorbat 85.

## Revendications

1. Procédé de fabrication d'une poudre pharmaceutique de préparation, le procédé comprenant :
a. l'atomisation d'une solution aqueuse par l'intermédiaire d'un sécheur à pulvérisation, la solution aqueuse comprenant :
i. 0,1 % à 2,0 % (m/v) d'un stabilisant thermique et 50 mg/mL à 180 mg/mL d'une protéine avec un rapport massique à ou entre 1 : 5 et 2 : 5, dans lequel le stabilisant thermique est sélectionné parmi : saccharose, tréhalose, mannitol, isoleucine, proline, ou des associations de ceux-ci, et la protéine est (a) une glycoprotéine contenant Fc sélectionnée parmi un anticorps ou une protéine de fusion de récepteur Fc, ou (b) un récepteur soluble, et
ii. 0,01 à 0,2 % (m/v) d'un surfactant non ionique sélectionné parmi le groupe constitué de : poly(oxyde d'éthylène) d'alkyle, polyglucosides d'alkyle, alcools gras, cocamide MEA, cocamide DEA, cocamide TEA, esters de sorbitane de polyoxyéthylène, poloxamères, poloxamère 188, poloxamère 407, polyéthylène-polypropylène glycol, et polyéthylène glycol,
dans lequel une température d'entrée du sécheur à pulvérisation est réglée à une température de 100 °C à 130 °C et une température de sortie du sécheur à pulvérisation est réglée à une température qui est en dessous du point d'ébullition d'eau et au-dessus de la température ambiante ; et
b. l'application de chaleur sur la solution aqueuse atomisée pour former la poudre pharmaceutique de préparation, dans lequel la poudre pharmaceutique de préparation n'est pas soumise à un séchage secondaire, dans lequel la poudre pharmaceutique de préparation comprend entre 3 % et 10 (m/m) d'eau.

2. Procédé selon la revendication 1, dans lequel la protéine de fusion de récepteur Fc est une protéine piège.

3. Procédé selon la revendication 1 ou 2, dans lequel la protéine est un récepteur soluble.

4. Procédé selon l'une quelconque des revendications 1 à 3, dans lequel la température de sortie du sécheur à pulvérisation est réglée à 55 °C.

5. Procédé selon la revendication 1, comprenant en outre :
la mise en suspension de la poudre pharmaceutique de préparation dans une solution organique comprenant un polymère biodégradable ; et
le séchage à pulvérisation de la suspension pour former une poudre pharmaceutique de préparation enrobée, dans lequel la poudre pharmaceutique de préparation n'est pas soumise à un séchage secondaire avant la formation de la poudre pharmaceutique de préparation enrobée.

6. Procédé de fabrication d'une poudre pharmaceutique de préparation, le procédé comprenant :
a. l'atomisation d'une solution aqueuse par l'intermédiaire d'un sécheur à pulvérisation, la solution aqueuse comprenant :
i. 0,1 % à 2,0 % (m/v) d'un stabilisant thermique et 50 mg/mL à 180 mg/mL d'une protéine à un rapport molaire inférieur à 300 moles du stabilisant thermique par mole de la protéine, dans lequel le stabilisant thermique est sélectionné parmi : saccharose, tréhalose, mannitol, isoleucine, proline, ou des associations de ceux-ci, dans lequel la protéine est (a) une glycoprotéine contenant Fc sélectionnée parmi un anticorps ou une protéine de fusion de récepteur Fc, ou (b) un récepteur soluble, et
ii. 0,01 à 0,2 % (m/v) d'un surfactant non ionique sélectionné parmi le groupe constitué de : poly(oxyde d'éthylène) d'alkyle, polyglucosides d'alkyle, alcools gras, cocamide MEA, cocamide DEA, cocamide TEA, esters de sorbitane de polyoxyéthylène, poloxamères, poloxamère 188, poloxamère 407, polyéthylène-polypropylène glycol, et polyéthylène glycol,
dans lequel une température d'entrée du sécheur à pulvérisation est réglée à une température de 100 °C à 130 °C et une température de sortie du sécheur à pulvérisation est réglée à une température qui est en dessous du point d'ébullition d'eau et au-dessus de la température ambiante ; et
b. l'application de chaleur sur la solution aqueuse atomisée pour former la poudre pharmaceutique de préparation, dans lequel la poudre pharmaceutique de préparation n'est pas soumise à un séchage secondaire, dans lequel la poudre pharmaceutique de préparation comprend entre 3 % et 10 % (m/m) d'eau.

7. Procédé selon la revendication 6, dans lequel la protéine de fusion de récepteur Fc est une protéine piège.

8. Procédé selon la revendication 6 ou 7, dans lequel la protéine est un récepteur soluble.

9. Procédé selon l'une quelconque des revendications 6 à 8, dans lequel la température de sortie du sécheur à pulvérisation est réglée à 55 °C.

10. Procédé selon la revendication 6, comprenant en outre :
la mise en suspension de la poudre pharmaceutique de préparation dans une solution organique comprenant un polymère biodégradable ; et
le séchage à pulvérisation de la suspension pour former une poudre pharmaceutique de préparation enrobée, dans lequel la poudre pharmaceutique de préparation n'est pas soumise à un séchage secondaire avant la formation de la poudre pharmaceutique de préparation enrobée.

11. Poudre pharmaceutique de préparation, comprenant :
60 % à 97 % (m/m) d'une glycoprotéine, et 3 % à 40 % (m/m) d'un stabilisant thermique, dans laquelle la glycoprotéine est un anticorps ou une protéine de fusion de récepteur Fc, et dans laquelle le stabilisant thermique est sélectionné parmi : saccharose, tréhalose, mannitol, isoleucine, proline, ou des associations de ceux-ci ;
0,01 à 0,2 % (m/v) d'un surfactant non ionique sélectionné parmi le groupe constitué de : poly(oxyde d'éthylène) d'alkyle, polyglucosides d'alkyle, alcools gras, cocamide MEA, cocamide DEA, cocamide TEA, esters de sorbitane de polyoxyéthylène, poloxamères, poloxamère 188, poloxamère 407, polyéthylène-polypropylène glycol, et polyéthylène glycol ; et
3% à 10 % (m/m) d'eau, dans laquelle la poudre pharmaceutique de préparation n'est pas soumise à un séchage secondaire, et dans laquelle le taux d'agrégation est inférieur à 5 % par mois Λ ½.

12. Poudre pharmaceutique de préparation selon la revendication 11, dans laquelle la protéine de récepteur Fc est une protéine piège.

13. Procédé selon l'une quelconque des revendications 1 à 10 ou poudre de préparation selon la revendication 11 ou 12, dans lequel ou laquelle le polyglucoside d'alkyle est sélectionné parmi : glucoside d'octyle et maltoside de décyle.

14. Procédé selon l'une quelconque des revendications 1 à 10 ou poudre de préparation selon la revendication 11 ou 12, dans lequel ou laquelle les alcools gras sont sélectionnés parmi : alcool cétylique et alcool oléylique.

15. Procédé selon l'une quelconque des revendications 1 à 10 ou poudre de préparation selon la revendication 11 ou 12, dans lequel ou laquelle l'ester de sorbitane de polyoxyéthylène est sélectionné parmi : polysorbate 20, polysorbate 28, polysorbate 40, polysorbate 60, polysorbate 65, polysorbate 80, polysorbate 81, et polysorbate 85.
